# EUROPEAN PATENT SPECIFICATION

(11) **EP 0 458 643 B1**
(45) Date of publication and mention of the grant of the patent: **29.11.1995**
(21) Application number: 91304697.5
(22) Date of filing: 23.05.1991
(51) Int. Cl.: C07C 67/347, C07C 69/757, C07C 69/78, C07D 307/32, C07D 317/24, C07C 67/29

(54) **Process for preparing an optically active cyclobutanone, an intermediate in the synthesis of an optically active cyclobutane nucleoside**
Verfahren zur Herstellung von optisch-aktivem Cyclobutanon, ein Zwischenprodukt in der Synthese von optisch-aktiven Cyclobutannukleosiden
Procédé pour la préparation d'une cyclobutanone optiquement active, un intermédiaire dans la synthèse d'un nucléoside cyclobutane optiquement actif

(30) Priority: 24.05.1990 US 528626
(43) Date of publication of application: 27.11.1991
(73) Proprietor: E.R. SQUIBB & SONS, INC., Princeton New Jersey 08543-4000 (US)
(72) Inventor: Ahmad, Saleem, Plainsboro, New Jersey 08536 (US)
(74) Representative: Thomas, Roger Tamlyn

(56) References cited:
- EP-A- 0 335 355
- EP-A- 0 358 154
- US-A- 3 317 586
- TETRAHEDRON LETT. vol. 32, no. 48, 25 November 1991, pages 6997 - 7000 S. AHMAD

## Description

The present invention provides a process for the preparation of the optically active cyclobutanone compound of formula 1 wherein R³ is a protecting group. 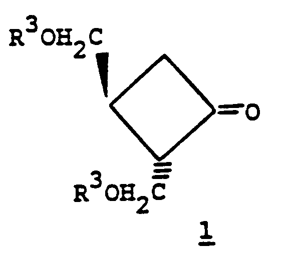
The invention also provides novel intermediates in the above process and a process for preparing these intermediates.

The optically active cyclobutanone compound of formula 1 is an intermediate in the synthesis of the optically active cyclobutane nucleoside analog 1R-(1α,2β,3α)-2-amino-9-[2,3-bis(hydroxymethyl)cyclobutyl]-1,9-dihydro-6H-purin-6-one, represented by formula 2. Antiviral activity is exhibited by compound 2 and its 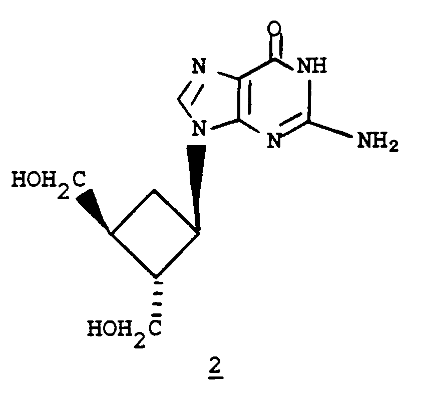
pharmaceutically acceptable salts. Unless otherwise stated, compounds 1 and 2 are optically active, and their absolute stereochemistry is (2S, 3S) and (1R, 2R, 3S), respectively as depicted in the above figures.

The preparation of compound 1 in optically active form and its conversion to optically active compound 2 has been described in Ichikawa, Y., et al., J. Chem. Soc. Chem. Commun. 1989, 1919-1921, in European patent application 358,154 published on March 14, 1990, and in European patent application 366,059 published on May 2, 1990.

The preparation of compound 1 in optically inactive form (i.e., as a racemic mixture) and its conversion to optically inactive 2, (±) - (1α,2β,3α)-2-amino-9-[2,3-bis(hydroxymethyl)cyclobutyl]-1,9-dihydro-6H-purin-6-one, has been described in Slusarchyk, W.A., et al. Tetrahedron Lett. 1989, 6453-6456; in European patent application 335,355 published on October 4, 1989; in Norbeck, D.W., et al. J. Med. Chem. 1990, 33, 1281 - 1285; and in European patent application 366,059 published on May 2, 1990.
An additional preparation of optically inactive compound 1 has been described in Honjo, M., et al. Chem. Pharm. Bull. 1989, 37, 1413-1415.

The process of the present invention is shown in the reaction scheme below: 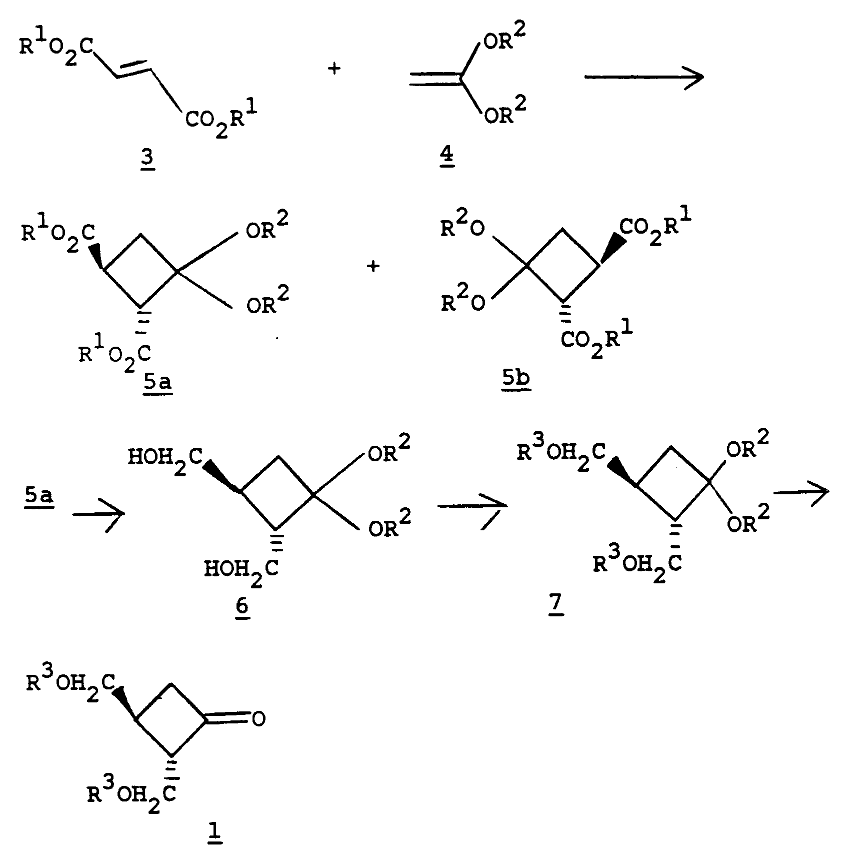
wherein R¹ is the group obtained by removal of the hydroxy group from the homochiral alcohol of the formula R¹OH and is a branched chain alkyl of 4 to 20 carbons, a substituted straight or branched chain alkyl of 1 to 20 carbons, a substituted cycloalkyl of 3 to 20 carbons, a bridged cycloalkyl of 6 to 20 carbons, a substituted bridged cycloalkyl of 6 to 20 carbons, a polycycloalkyl of 7 to 20 carbons, a substituted polycycloalkyl of 7 to 20 carbons wherein said substituents are one or more, preferably one, two or three, selected from lower alkyl of 1 to 5 carbons, halo, lower alkoxy of 1 to 5 carbons, 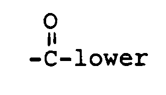
alkoxy of 1 to 5 carbons 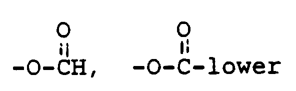
alkyl of 1 to 5 carbons and phenyl, a lactone of 4 to 6 carbons, a substituted lactone of 4 to 6 carbons wherein said lactone substituents are one or more, preferably one or two, selected from lower alkyl of 1 to 5 carbons, halo, lower alkoxy of 1 to 5 carbons, and phenyl, 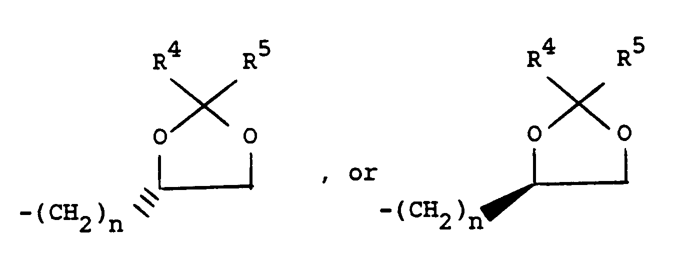
wherein n is an integer from 1 to 4, and R⁴ and R⁵ are independently selected from hydrogen and lower alkyl of 1 to 5 carbons;
R² is lower alkyl of 1 to 5 carbons or both R² groups are joined together by an alkylene group of 2 or 3 carbons; and
R³ is a protecting group.

Compounds 5a and 5b are diasteromeric relative to each other, and the absolute stereochemistry at the cyclobutane 1- and 2-positions is (1S, 2R) for compound 5a and (1R, 2S), for compound 5b as depicted in the figures of the above reaction scheme. Unless otherwise stated, compounds 6 and 7 are optically active, and their absolute stereochemistry is (1S, 2S), as depicted in the figures of the above reaction scheme.

The preparation of compounds 6 and 7 in optically inactive form has previously been described in Slusarchyk, W. A., et al. Tetrahedron Lett. 1989, 6453 - 6456 and European patent application 335,355 published on October 4, 1989.

The term "lower alkyl" refers to both straight and branched chain groups which contain from 1 to 5 carbons. Similarly, the term "lower alkoxy" refers to such lower alkyl groups attached to an oxygen. The term "halo" refers to Br, Cl, F and I.

Suitable R³ protecting groups include hindered silyl groups such as t-butyldiphenylsilyl and triisopropylsilyl, acyl groups such as acetyl and benzoyl, benzyl and substituted benzyl groups such as 4-methoxybenzyl and 3,4-dimethoxybenzyl.

Suitable R¹ groups include 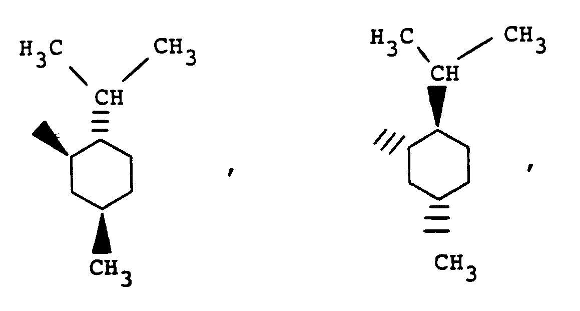 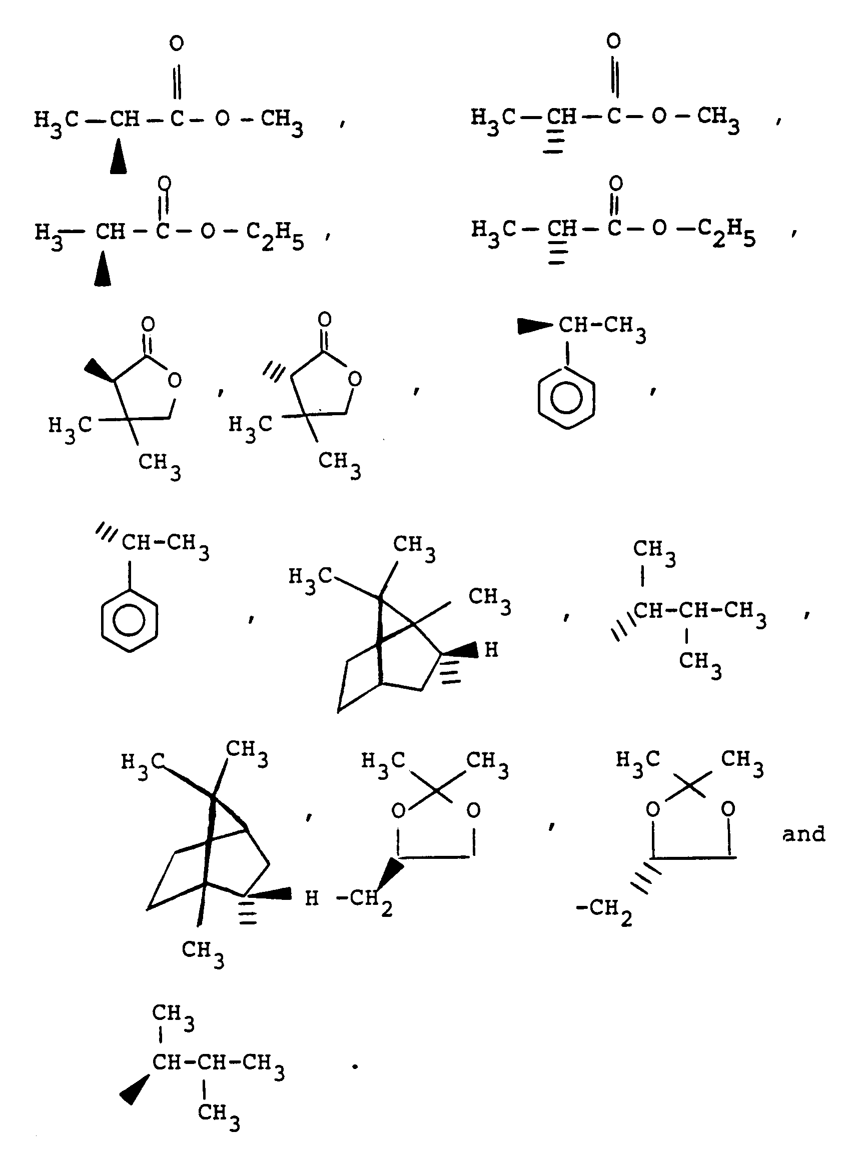
These R¹ groups are obtained from the optically active alcohols (+)- or (-)-menthol, (+)- or (-)-methyl- or ethyl lactate, (+)- or (-)-pantolactone, (+)- or (-)-α-phenethyl alcohol, (+)- or (-)-borneol, (+)- or (-)-α,β isopropylideneglycerol, (+)- or (-)-3-methyl-2-butanol and the like (i.e., wherein R¹OH is (+)- or (-)-menthol, (+)- or (-)-methyl or ethyl lactate, (+)- or (-)pantolactone, (+)- or (-)-α-phenethyl alcohol, (+)- or (-)-borneol, (+)- or (-)-α,β-isopropylidene-glycerol, (+)- or (-)-3-methyl-2-butanol by removal of the hydroxy group.

Preferred features of the invention will now be described.

Preferred R² groups are methyl and ethyl.

The compound of formula 5a is prepared by reaction of a compound of formula 3 with a compound of formula 4 in the presence of a Lewis acid. Examples of such Lewis acids include aluminum compounds such as diethylaluminum chloride, diisobutylaluminum chloride, ethylaluminum dichloride, isopropoxyaluminum dichloride, aluminum trichloride and the like, boron compounds such as boron trifluoride, boron trichloride and the like, titanium compounds such as titanium tetrachloride, dichlorodiisopropoxytitanium and the like, and tin compounds such as tin tetrachloride, tin trichloride and the like. The compound of formula 3 and the compound of formula 4 are used in a proportion wherein the amount of compound 4 is 0.1 to 5 equivalents per equivalent of compound 3. The Lewis acid catalyst is used in an amount of 0.5 to 5 equivalents per equivalent of compound 3. The reaction is carried out in a solvent such as methylene chloride, toluene, hexane, petroleum ether, mixtures of toluene and hexane and the like. The reaction mixture is stirred for 1 minute to 24 hours at a temperature of -100°C to 25°C. It should be noted that when the Lewis acid is titanium tetrachloride, tin tetrachloride, boron trifluoride etherate or boron tribromide then R¹ cannot be derived from (-)-menthol and R² cannot be methyl. In addition to 5a, varying quantities of its diastereomer 5b may also be produced in the reaction. The relative amounts of 5a and 5b produced in the reaction will depend upon the reactants, reagents, and conditions of the reaction; in particular the relative amounts of 5a and 5b will depend upon the absolute stereochemistry of the R¹ group chosen. The crude 5a obtained from the reaction can be purified by crystallization or by chromatography; 5a is a novel compound.

Preferably, the R¹ group for the compound of formula 3 is derived from (-)-menthol, i.e., R¹ is 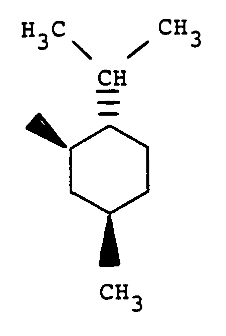
and the Lewis acid is a di(lower alkyl) aluminum chloride, particularly where each lower alkyl group is of 2 to 4 carbons. When the R¹ group for the compound of formula 3 is derived from (-)-menthol and the Lewis acid is a di(lower alkyl) aluminum chloride, the amount of compound 4 employed for the reaction is preferably 1 to 2 equivalents per equivalent of 3, the amount of Lewis acid employed is preferably 1.5 to 2.5 equivalents per equivalent of 3,the reaction mixture is stirred preferably for about 5 minutes to 2 hours at a temperature of preferably about -80°C to -40°C.

Most preferably, the R¹ group for the compound of formula 3 is derived from (-)-menthol, the Lewis acid is diethylaluminum chloride or diisobutylaluminum chloride and R² is methyl. For example, when compound 3, wherein the group R¹ is derived from (-)-menthol, is reacted with 1.1 equivalents of the compound of formula 4, wherein R² is methyl, in the presence of 2 equivalents of diisobutylaluminum chloride at about -78°C in toluene for about 30 minutes, 5a is obtained in high yield and in large excess relative to the diastereomer 5b. The resulting crude 5a is purified by chromatography on silica gel, or by crystallization from methanol or methanol-water.

A compound of formula 3 wherein R¹ is as defined above can be obtained commercially (e.g., the compound of formula 3 wherein R¹ is the group derived from the optically active alcohol (-)-menthol can be obtained from Aldrich Chemical Company) or can be readily prepared by methods known in the art (see e.g., Heathcock, C.H., et al., J. Med. Chem. 1989, 32, 197-202; Scharf, H. D., et al. Chem Ber. 1986, 119, 3492-3497; Helmchen, G. et al., DE 3702084). Compounds of formula 4 wherein R² is as defined above are either commercially available (e.g. Wiley Organics Inc.) or can be readily prepared by methods known in the art (see e.g., Orqanic Synthesis Collective Volume *III*, P. 506; S.M. McElvain J. Amer. Chem. Soc. 1955, 77, 5601-5606). The Lewis acids are commercially available or can be prepared by methods known in the art.

The compound of formula 6 is prepared by reacting a compound of formula 5a with a reducing agent such as lithium aluminum hydride, lithium borohydride, and the like, in a solvent such as tetrahydrofuran, diethyl ether, and the like. The reaction mixture is stirred for 5 minutes to 24 hours, preferably for 30 minutes to 4 hours at a temperature of 0°C to the reflux temperature of the solvent. The compound of formula 6 is isolated and purified by methods known in the art.

A compound of formula 7 wherein R³ is a protecting group is prepared by reacting a compound of formula 6 with the corresponding protecting group precursor. Suitable protecting groups R³ include hindered silyl groups (such as t-butyldiphenylsilyl or triisopropylsilyl), benzyl or substituted benzyl groups, acyl groups (such as acetyl or benzoyl, preferably benzoyl) and the like. A compound of formula 7 wherein R³ is a hindered silyl group is prepared by treating a compound of formula 6 with the appropriate silyl reagent e.g., the corresponding tri(hydrocarbon)-silyl chloride, in a solvent such as dimethylformamide or tetrahydrofuran in the presence of a base such as triethylamine or imidazole. A compound of formula 7 wherein R³ is a benzyl or substituted benzyl is prepared by treating a compound of formula 6 with a benzyl halide or a substituted benzyl halide in a solvent such as tetrahydrofuran or dimethylformamide in the presence of a suitable base such as sodium hydride. A compound of formula 7 wherein R³ is an acyl group such as acetyl or benzoyl is prepared by treating a compound of formula 6 with the corresponding acyl anhydride or acyl halide, preferably benzoyl chloride or benzoic anhydride, in a solvent such as pyridine or tetrahydrofuran/triethylamine, or ethyl acetate/triethylamine, preferably ethyl acetate/triethylamine. Optionally, a catalyst such as 4,4-dimethylaminopyridine is added to the reaction mixture. The benzoylation reaction is carried out at -10°C to 35°C, preferably at -5°C to 25°C, 1/4 hour to 48 hours, preferably 1/2 hour to 24 hours. Water is added to the reaction mixture, the mixture is stirred, and the product is extracted and optionally purified e.g. by chromatography.

A compound of formula 1 is prepared by treatment of a compound of formula 7 with an acid catalyst such as sulfuric acid, hydrochloric acid, p-toluenesulfonic acid, and the like, preferably sulfuric acid, in a solvent or solvent mixture such as water, water-acetonitrile, water-dioxane, acetone and the like, preferably water-acetonitrile. The reaction mixture is stirred at 0°C to 60°C, preferably at 15°C to 30°C for 1/2 hour to 48 hours, preferably for 2 hours to 24 hours. The reaction mixture is neutralized and the product is extracted and optionally purified by e.g. chromatography or crystallization.

The following examples are specific embodiments of the invention.

### Example 1

### (1S-trans)-3,3-Dimethoxy-1,2-cyclobutanedicarboxylic acid, di-(-)-menthyl ester

Di-(-)-menthylfumarate (100 g) was dissolved in 400 ml dry toluene and cooled to -75°C under argon. To this solution was added with stirring diisobutylaluminum chloride (99.5 ml) over 15 minutes. The resulting orange mixture was stirred at -75°C for 15 minutes and 1,1-dimethoxyethylene (24.7 g) was added over 15 minutes. After stirring the reaction mixture for 10 minutes at -78°C, methanol (30 ml) was added over 15 minutes and the mixture was stirred for 30 minutes. Hexane (250 ml) was added over 5 minutes followed by the addition of 20% aqueous sodium hydroxide (40 ml) over 15 minutes at -60°C to -40°C. The reaction mixture was slowly (over 45 minutes) allowed to warm to 10°C and anhydrous magnesium sulfate (40 g) was added. The mixture was allowed to come to room temperature, was filtered, and the filtrate was concentrated *in vacuo* to afford an oil (119.5 g) which solidified under vacuum. The crude product was recrystallized from methanol-water (95:5) to afford 102g of the title compound (isomerically pure as judged by HPLC) as a white solid, m.p. 89°C, [α]_{D} =-28.5° (c = 1, CHCl₃).

Alternatively, the title compound can be prepared by using diethylaluminum chloride instead of diisobutylaluminum chloride, as described below.

A solution of diethylaluminum chloride (1M in hexane, 5.1 ml) was added dropwise to a stirred solution of di-(-)-menthyl fumarate (1.0 g) in 5 ml toluene under nitrogen at -78°C. The reaction mixture was stirred at that temperature for 15 minutes, followed by the addition of 0.247 g 1,1-dimethoxyethylene. The reaction mixture was stirred at -78°C for 30 minutes, then was carefully added to a mixture of 50 ml hexane and 20 ml saturated aqueous sodium bicarbonate solution. The organic phase was washed with additional saturated sodium bicarbonate solution (2 x 20 ml), water (3 x 20 ml), and was dried (magnesium sulfate) and concentrated *in vacuo* giving 1.23 g of a thick oil. The crude mixture was recrystallized from methanol-water (95:5) affording 0.98 g of the title compound pure by HPLC and NMR.

### Example 2

### (1S-trans)-3,3-Dimethoxy-1,2-cyclobutanedimethanol

A solution of (1S-trans)-3,3-dimethoxy-1,2-cyclobutanedicarboxylic acid, di-(-)-menthyl ester (3.5 g) in 15 ml dry tetrahydrofuran was added dropwise over 5 minutes to a suspension of 420 mg of lithium aluminum hydride in 73 ml of dry tetrahydrofuran at room temperature and under argon. The mixture was heated at 55°C for 1 hour, after which no starting material was observed by thin layer chromatography. To the reaction mixture, cooled to 5°C, was added sequentially 420 »l water, 420 »l of 15% aqueous sodium hydroxide, and 1.28 ml of water. The resulting suspension was stirred for 15 minutes at room temperature, then ca. 5g of anhydrous magnesium sulfate was added, and stirring was continued an additional 0.5 hour. The solids were removed by filtration through Celite, and the clear, colorless filtrate was concentrated to afford 3.7 g of a semi-solid residue (a mixture of the title compound and (-)-menthol). This residue was dissolved in a mixture of 35 ml water, 3.5 ml methanol and 20 ml heptane. The organic layer was separated and the aqueous layer was extracted with additional heptane (3 x 10 ml). The combined organic fractions were washed once with 5 ml of water. The combined aqueous fractions were concentrated *in vacuo* to ca. 5 ml, saturated with sodium chloride, and extracted with ethyl acetate (2 x 20 ml). The combined ethyl acetate extracts were dried over anhydrous magnesium sulfate and concentrated to afford 1.33 g of the title compound as a clear colorless oil.
¹HNMR (CDCL₃): δ1.69 (ddd, 1H, J = 1.2, 7.8, 12.3 Hz, H-4); 2.1 (m, 1H, H-3); 2.35 (m, 2H, H-2 and H-4); 2.64 (s, 2H, 2 x OH); 3.184 (s, 3H, OCH₃); 3.188 (s, 3H, OCH₃); 3.51 (dd, 1H, J = 8.8, 10.5 Hz) and 3.75 (m, 3H) [2 x OCH₂].

Alternatively, the above-described crude semi-solid mixture of the title compound and (-)-menthol can be treated as follows. The crude mixture resulting from the lithium aluminum hydride reduction of 17.5 g of (1S-*trans*)-3,3-dimethoxy-1,2-cyclobutanedicarboxylic acid, di-(-)-menthyl ester was dissolved in 100 ml of heptane and was washed with water (4 x 50 ml). The combined aqueous fractions were washed with 25 ml of heptane, then were saturated with ammonium sulfate (45g) and were extracted with ethyl acetate (4 x 50 ml). The combined ethyl acetate extracts were dried over anhydrous magnesium sulfate and concentrated to afford 6.3 g of the title compound as a colorless oil.

### Example 3

### (1S-trans)-3,3-Dimethoxy-1,2-cyclobutanedimethanol, dibenzoate ester

To a solution of 300 mg of (1S-trans)-3,3-dimethoxy-1,2-cyclobutanedimethanol in 3.4 ml of dry pyridine at 5°C under argon was added dropwise 494 »l of benzoyl chloride. The mixture was stirred with cooling for an additional 1.5 hours after which 100 »l water was added. The reaction mixture was diluted with ethyl acetate and washed with 10% aqueous hydrochloric acid, brine, saturated sodium bicarbonate and brine. The organic solution was dried over anhydrous magnesium sulfate and concentrated to afford 642 mg of the title compound as a clear, colorless oil. An analytical sample of the title compound was prepared by semi-preparative HPLC, [α]_{D} =54.3° (c =0.94, CHCl₃).

Alternatively, a solution of 147.7 g. of (1S-trans)-3,3-dimethoxy-1,2-cyclobutanedimethanol in 900 ml. of ethyl acetate, chilled in an ice bath to an internal temperature of 8°C, was treated with 410 g. of benzoic anhydride, 211 g. of triethylamine, and 6.5 g. of 4,4-dimethylaminopyridine. The stirred mixture was allowed to come to room temperature over 15 hours. The mixture was then treated with 45 ml. of water and stirred at room temperature for 6 hours. The mixture was diluted with 1 L of ethyl acetate and washed with water (2 x 1 L), 1N HCl (2 x 0.5 L), water (0.5 L), 10% sodium bicarbonate (2 x 0.5 L), and brine (0.5L). The organic solution was dried over anhydrous magnesium sulfate and was concentrated to afford 309.2 g. of the title compound.

### Example 4

### (2S-trans)-2,3-Bis[(benzoyloxy)methyl]-cyclobutanone

A solution of (1S-trans)-3,3-dimethoxy-1,2-cyclobutanedimethanol, dibenzoate ester (312 mg) in 12 ml of acetonitrile and 3.8 ml of 0.5N aqueous sulfuric acid was stirred at room temperature overnight. An additional 1.9 ml of 0.5N aqueous sulfuric acid was added and stirring was continued for an additional 6 hours, after which no starting material remained by thin layer chromatography. The solution was diluted with ethyl acetate and was washed with brine, saturated aqueous sodium bicarbonate and brine. The organic solution was dried over anhydrous magnesium sulfate and was concentrated to afford 245 mg of the title compound as a white solid. An analytical sample of the title compound was recrystallized from methylene chloride-ether, m.p. 95.5 - 97°C, [α]_{D} = 24.1° (c = 1.31, CHCl₃).

## Claims (Claims for the following Contracting State(s): AT, BE, CH, DE, DK, FR, GB, GR, IT, LI, LU, NL, SE,)

1. A process for the preparation of the optically active compound 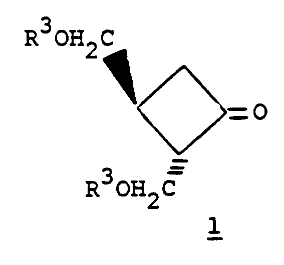 wherein R³ is a protecting group which comprises:
(a) reacting a compound of the formula 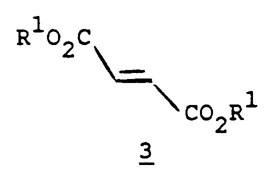 with a compound of the formula 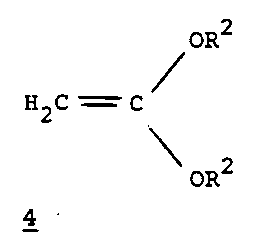 in the presence of a Lewis acid to yield a mixture of diastereomeric isomers of the formulae: 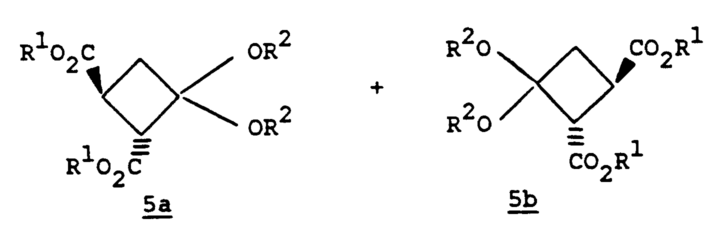 wherein R¹ is the group obtained by removal of the hydroxy group from the homochiral alcohol of the formula R¹OH and is a branched chain alkyl of 4 to 20 carbons, a substituted straight or branched chain alkyl of 1 to 20 carbons, a substituted cycloalkyl of 3 to 20 carbons, a bridged cycloalkyl of 6 to 20 carbons, a substituted bridged cycloalkyl of 6 to 20 carbons, a polycycloalkyl of 7 to 20 carbons, a substituted polycycloalkyl of 7 to 20 carbons wherein said substituents are one or more selected from lower alkyl of 1 to 5 carbons, halo, lower alkoxy of 1 to 5 carbons, 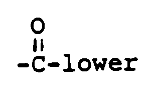 alkoxy of 1 to 5 carbons 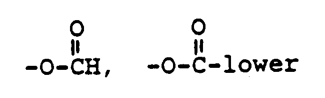 alkyl of 1 to 5 carbons and phenyl, a lactone of 4 to 6 carbons, a substituted lactone of 4 to 6 carbons wherein said lactone substituents are one or more selected from lower alkyl of 1 to 5 carbons, halo, lower alkoxy of 1 to 5 carbons, and phenyl, 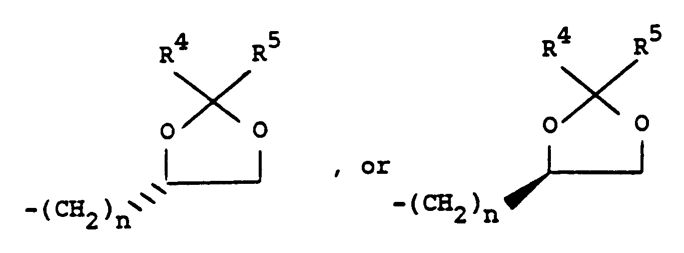 wherein n is an integer from 1 to 4, and R⁴ and R⁵ are independently selected from hydrogen and lower alkyl of 1 to 5 carbons;
R² is lower alkyl of 1 to 5 carbons or both R² groups are joined together by an alkylene group of 2 or 3 carbons; with the proviso that when R¹ is 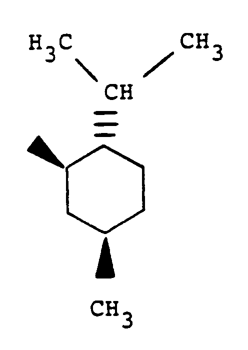 and R² is methyl, then the Lewis acid cannot be titanium tetrachloride, tin tetrachloride, boron trifluoride etherate or boron tribromide;
b) obtaining pure compound 5a by crystallization or chromatography;
c) reacting compound 5a with a reducing agent to form a compound of the formula 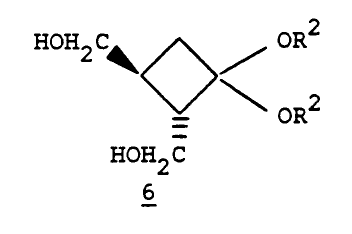
d) protecting the hydroxy groups of compound 6 with a group (R³) to form a compound of the formula 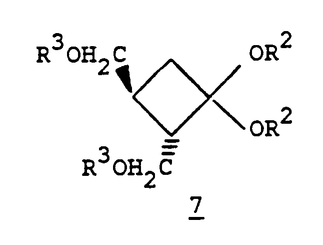 and
e) hydrolysing a compound 7 using an acid catalyst to form the compound of formula 1.

2. The process according to Claim 1 wherein the Lewis acid is selected from diethylaluminum chloride, diisobutylaluminum chloride, ethylaluminum dichloride, isopropoxyaluminum dichloride, aluminum trichloride, boron trifluoride, boron trichloride, titanium tetrachloride, dichlorodiisopropoxytitanium, tin tetrachloride and tin trichloride.

3. The process according to Claim 1 wherein the Lewis acid is a di(lower alkyl)aluminum chloride wherein each lower alkyl group in the Lewis acid is of 2 to 4 carbons.

4. The process according to Claim 1, 2 or 3 wherein R¹ is selected from

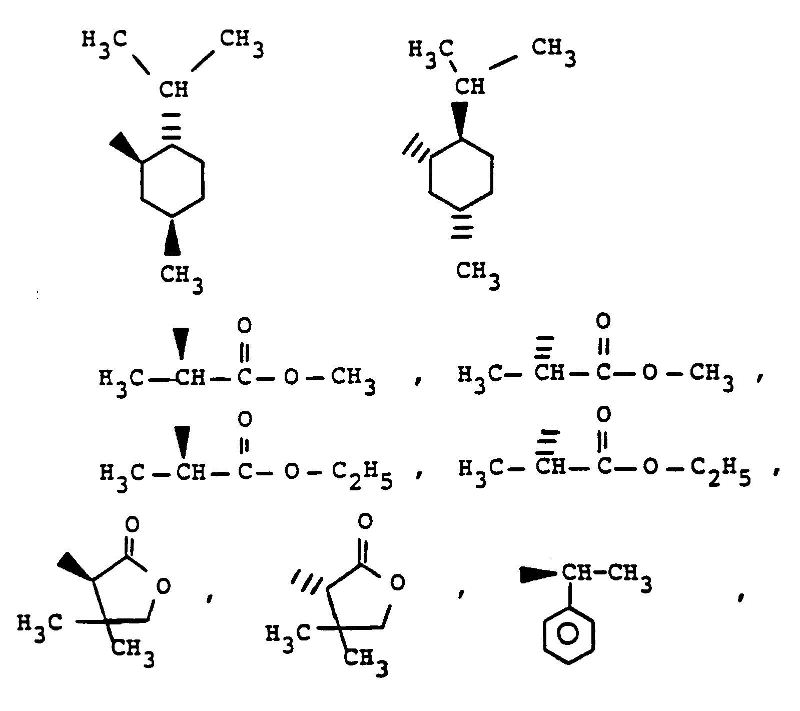

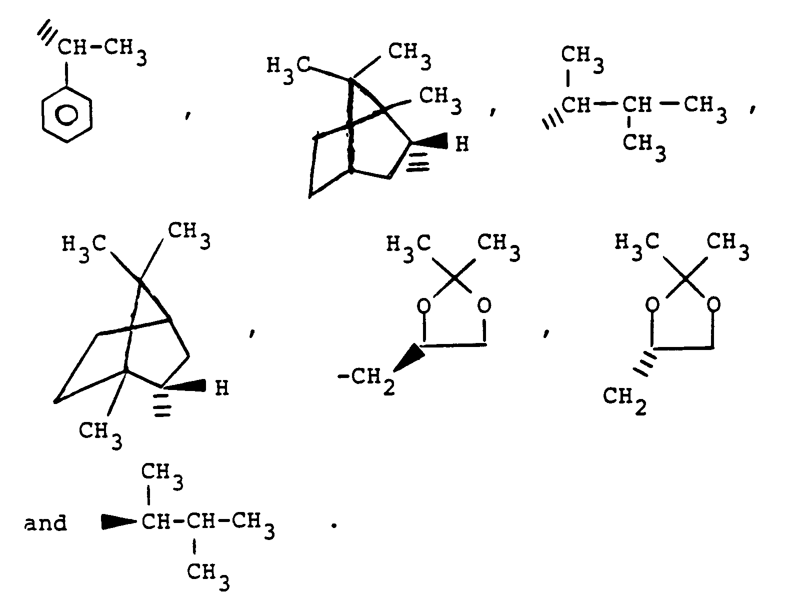

5. The process according to Claim 1, 2 or 3 wherein R¹ is 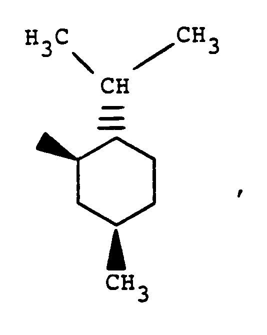 and
R² is methyl.

6. The process according to any one of Claims 1-5 wherein compound 4 is present in the amount of 0.1 to 5.0 equivalents per equivalent of compound 3 and the Lewis acid is present in an amount of 0.5 to 5.0 equivalents per equivalent of compound 3 and wherein the reaction in step (a) is stirred from 1 minute to 24 hours at a temperature of -100°C to 25°C.

7. The process according to Claim 1 wherein R¹ is 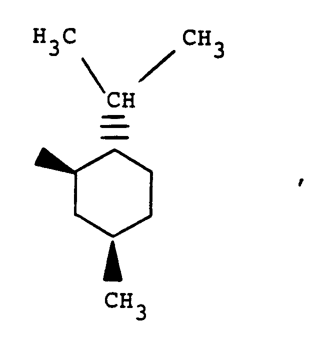 R² is methyl,
the Lewis acid is diisobutylaluminum chloride,
compound 4 is present in the amount of 1.0 to 2 equivalents per equivalent of compound 3 and the Lewis acid is present in an amount of 1.5 to 2.5 equivalents per equivalent of compound 3,
the reducing agent of step (c) is lithium aluminum hydride, and
the protecting group in step (d) is benzoyl.

8. A compound of the formula 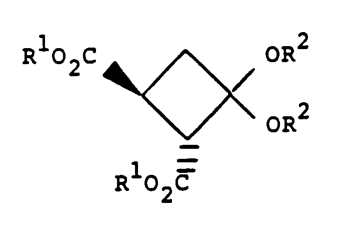 wherein the absolute stereochemistry at the cyclobutane is (1S, 2R) and R¹ is the group obtained by removal of the hydroxy group from the homochiral alcohol of the formula R¹OH and is a branched chain alkyl of 4 to 20 carbons, a substituted straight or branched chain alkyl of 1 to 20 carbons, a substituted cycloalkyl of 3 to 20 carbons, a bridged cycloalkyl of 6 to 20 carbons, a substituted bridged cycloalkyl of 6 to 20 carbons, a polycycloalkyl of 7 to 20 carbons, a substituted polycycloalkyl of 7 to 20 carbons wherein said substituents are one or more selected from lower alkyl of 1 to 5 carbons, halo, lower alkoxy of 1 to 5 carbons, 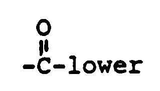 alkoxy of 1 to 5 carbons, 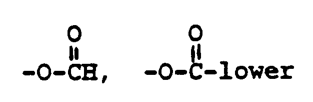 alkyl of 1 to 5 carbons and phenyl, a lactone of 4 to 6 carbons, a substituted lactone of 4 to 6 carbons wherein said lactone substituents are one or more selected from lower alkyl of 1 to 5 carbons, halo, lower alkoxy of 1 to 5 carbons, and phenyl, 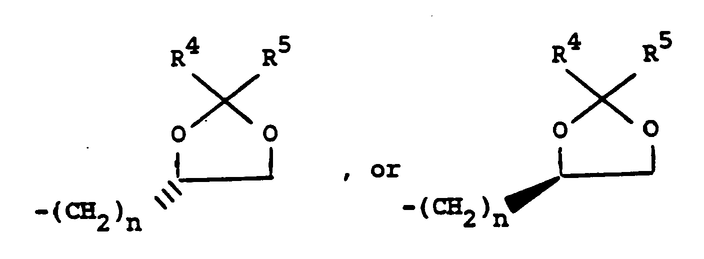 wherein n is an integer from 1 to 4, and R⁴ and R⁵ are independently selected from hydrogen and lower alkyl of 1 to 5 carbons; and
R² is lower alkyl of 1 to 5 carbons or both R² groups are joined together by an alkylene group of 2 or 3 carbons.

9. A compound according to Claim 8 wherein R¹ is selected from

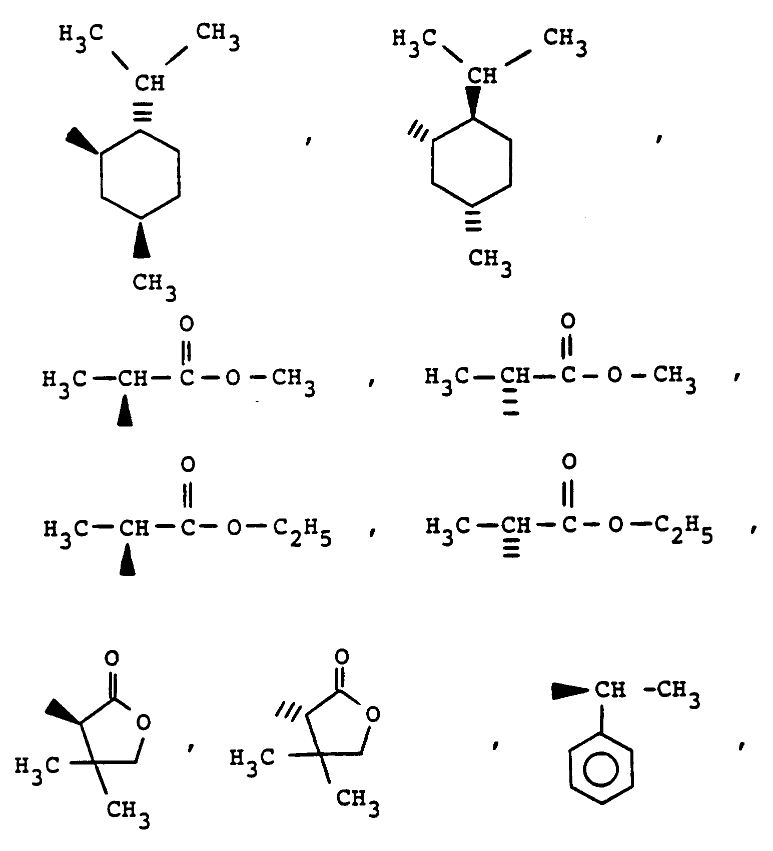

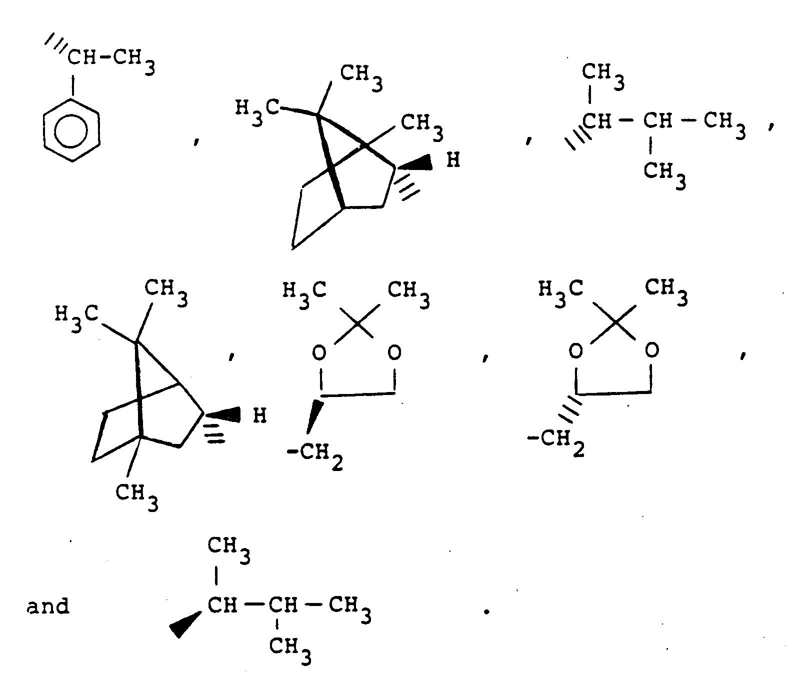

10. A compound according to Claim 8 wherein R¹ is 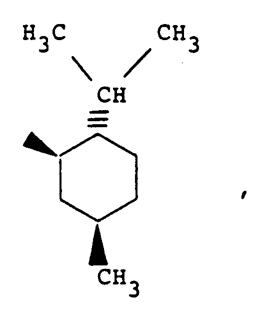 R² is methyl having the name, (1S-trans)-3,3-dimethoxy-1,2-cyclobutanedicarboxylic acid, di-(-)-menthyl ester.

## Claims (Claims for the following Contracting State(s): ES)

1. A process for the preparation of the optically active compound 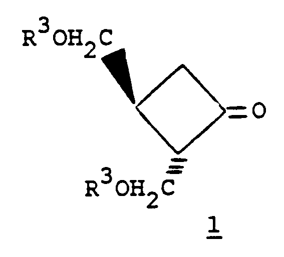 wherein R³ is a protecting group which comprises:
a) reacting a compound of the formula 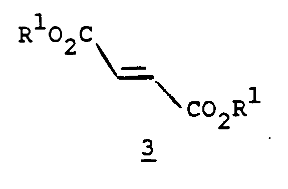 with a compound of the formula 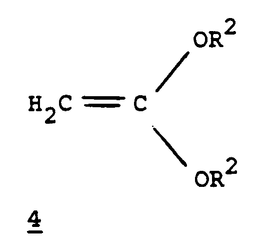 in the presence of a Lewis acid to yield a mixture of diastereomeric isomers of the formulae: 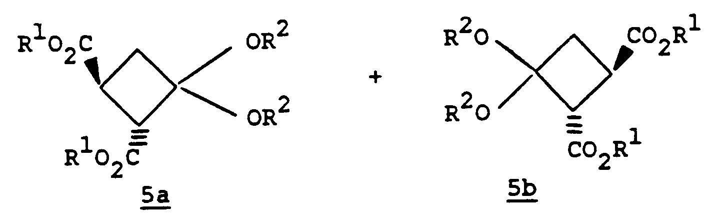 wherein R¹ is the group obtained by removal of the hydroxy group from the homochiral alcohol of the formula R¹OH and is a branched chain alkyl of 4 to 20 carbons, a substituted straight or branched chain alkyl of 1 to 20 carbons, a substituted cycloalkyl of 3 to 20 carbons, a bridged cycloalkyl of 6 to 20 carbons, a substituted bridged cycloalkyl of 6 to 20 carbons, a polycycloalkyl of 7 to 20 carbons, a substituted polycycloalkyl of 7 to 20 carbons wherein said substituents are one or more selected from lower alkyl of 1 to 5 carbons, halo, lower alkoxy of 1 to 5 carbons, 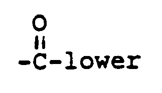 alkoxy of 1 to 5 carbons 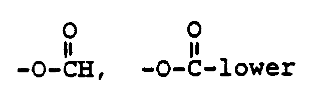 alkyl of 1 to 5 carbons and phenyl, a lactone of 4 to 6 carbons, a substituted lactone of 4 to 6 carbons wherein said lactone substituents are one or more selected from lower alkyl of 1 to 5 carbons, halo, lower alkoxy of 1 to 5 carbons, and phenyl, 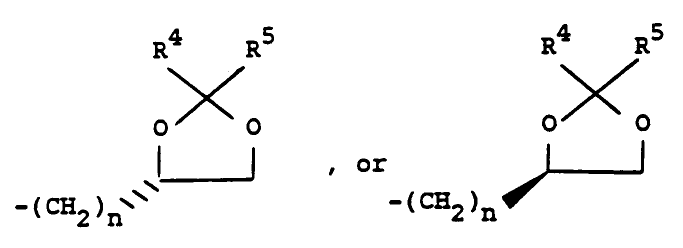 wherein n is an integer from 1 to 4, and R⁴ and R⁵ are independently selected from hydrogen and lower alkyl of 1 to 5 carbons;
R² is lower alkyl of 1 to 5 carbons or both R² groups are joined together by an alkylene group of 2 or 3 carbons; with the proviso that when R¹ is 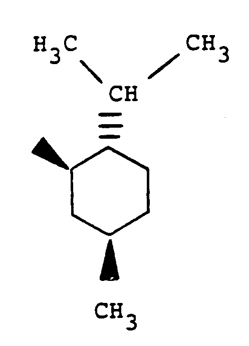 and R² is methyl, then the Lewis acid cannot be titanium tetrachloride, tin tetrachloride, boron trifluoride etherate or boron tribromide;
b) obtaining pure compound 5a by crystallization or chromatography;
c) reacting compound 5a with a reducing agent to form a compound of the formula 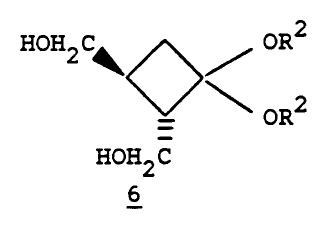
d) protecting the hydroxy groups of compound 6 with a group (R³) to form a compound of the formula 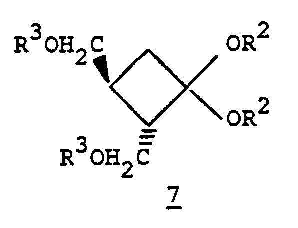
e) hydrolysis of a compound of formula 7 using an acid catalyst to form the compound of formula 1.

2. The process according to Claim 1 wherein the Lewis acid is selected from diethylaluminum chloride, diisobutylaluminum chloride, ethylaluminum dichloride, isopropoxyaluminum dichloride, aluminum trichloride, boron trifluoride, boron trichloride, titanium tetrachloride, dichlorodiisopropoxytitanium, tin tetrachloride and tin trichloride.

3. The process according to Claim 1 wherein the Lewis acid is a di(lower alkyl)aluminum chloride wherein each lower alkyl group in the Lewis acid is of 2 to 4 carbons.

4. The process according to Claim 1 wherein R¹ is selected from

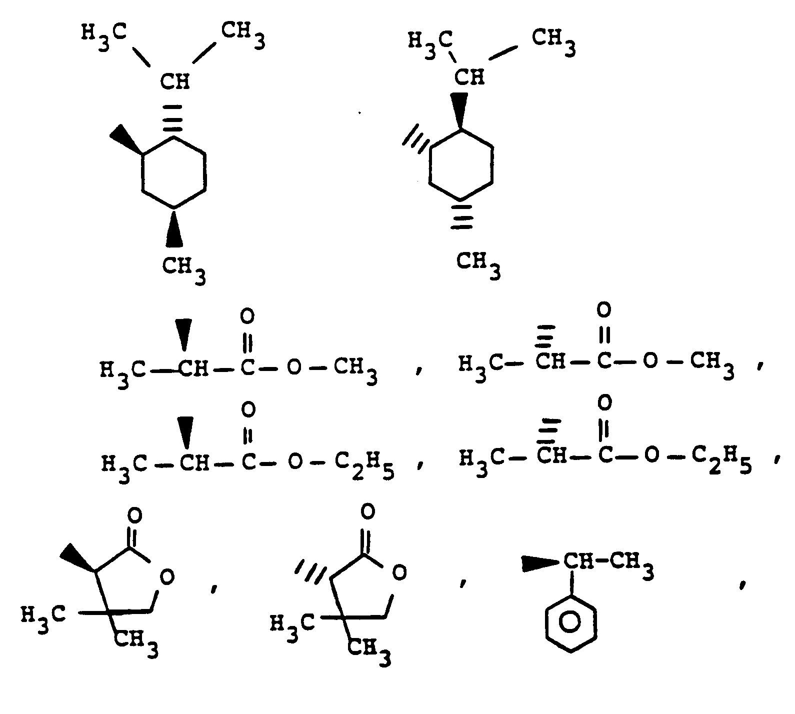

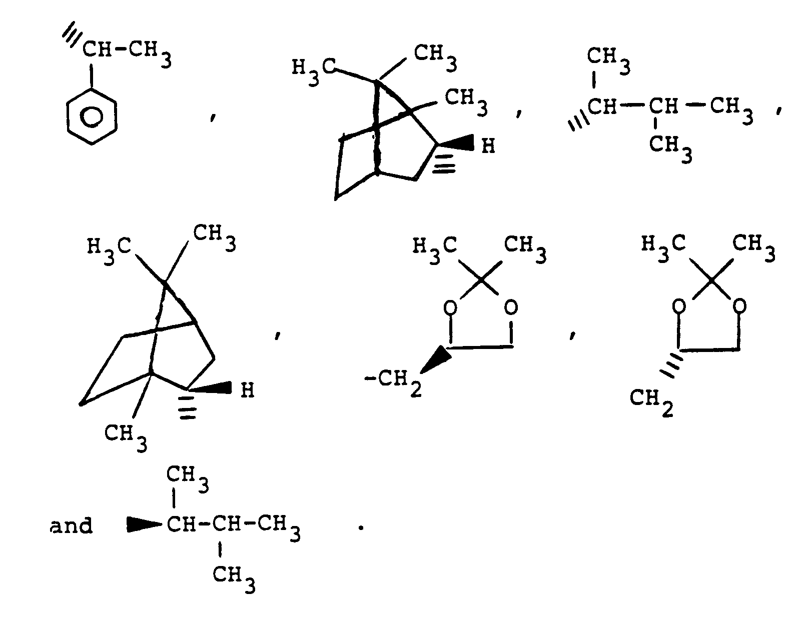

5. The process according to Claim 1 wherein R¹ is 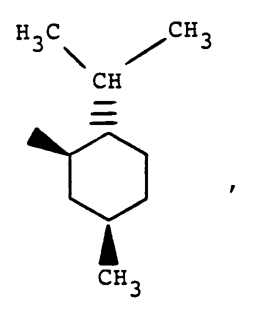 R² is methyl.

6. The process according to Claim 1 wherein compound 4 is present in the amount of 0.1 to 5.0 equivalents per equivalent of compound 3 and the Lewis acid is present in an amount of 0.5 to 5.0 equivalents per equivalent of compound 3 and wherein the reaction in step (a) is stirred from 1 minute to 24 hours at a temperature of -100°C to 25°C.

7. The process according to Claim 1 wherein R¹ is 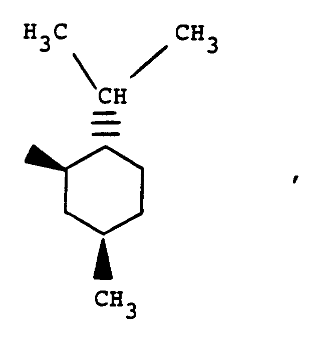 R² is methyl,
the Lewis acid is diisobutylaluminum chloride,
compound 4 is present in the amount of 1.0 to 2 equivalents per equivalent of compound 3 and the Lewis acid is present in an amount of 1.5 to 2.5 equivalents per equivalent of compound 3,
the reducing agent of step (c) is lithium aluminum hydride, and
the protecting group in step (d) is benzoyl.

8. Use of a compound of the formula 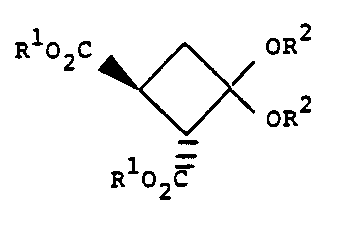 wherein the absolute stereochemistry at the cyclobutane is (1S, 2R) and R¹ is the group obtained by removal of the hydroxy group from the homochiral alcohol of the formula R¹OH and is a branched chain alkyl of 4 to 20 carbons, a substituted straight or branched chain alkyl of 1 to 20 carbons, a substituted cycloalkyl of 3 to 20 carbons, a bridged cycloalkyl of 6 to 20 carbons, a substituted bridged cycloalkyl of 6 to 20 carbons, a polycycloalkyl of 7 to 20 carbons, a substituted polycycloalkyl of 7 to 20 carbons wherein said substituents are one or more selected from lower alkyl of 1 to 5 carbons, halo, lower alkoxy of 1 to 5 carbons, 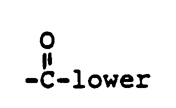 alkoxy of 1 to 5 carbons, 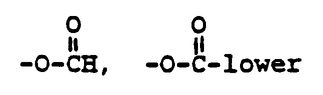 alkyl of 1 to 5 carbons and phenyl, a lactone of 4 to 6 carbons, a substituted lactone of 4 to 6 carbons wherein said lactone substituents are one or more selected from lower alkyl of 1 to 5 carbons, halo, lower alkoxy of 1 to 5 carbons, and phenyl, 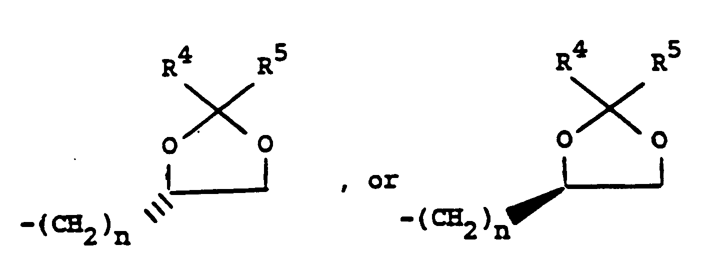 wherein n is an integer from 1 to 4, and R⁴ and R⁵ are independently selected from hydrogen and lower alkyl of 1 to 5 carbons; and
R² is lower alkyl of 1 to 5 carbons or both R² groups are joined together by an alkylene group of 2 or 3 carbons, for the preparation of a compound of formula 1 as defined in Claim 1.

9. Use according to Claim 8 wherein R¹ is selected from

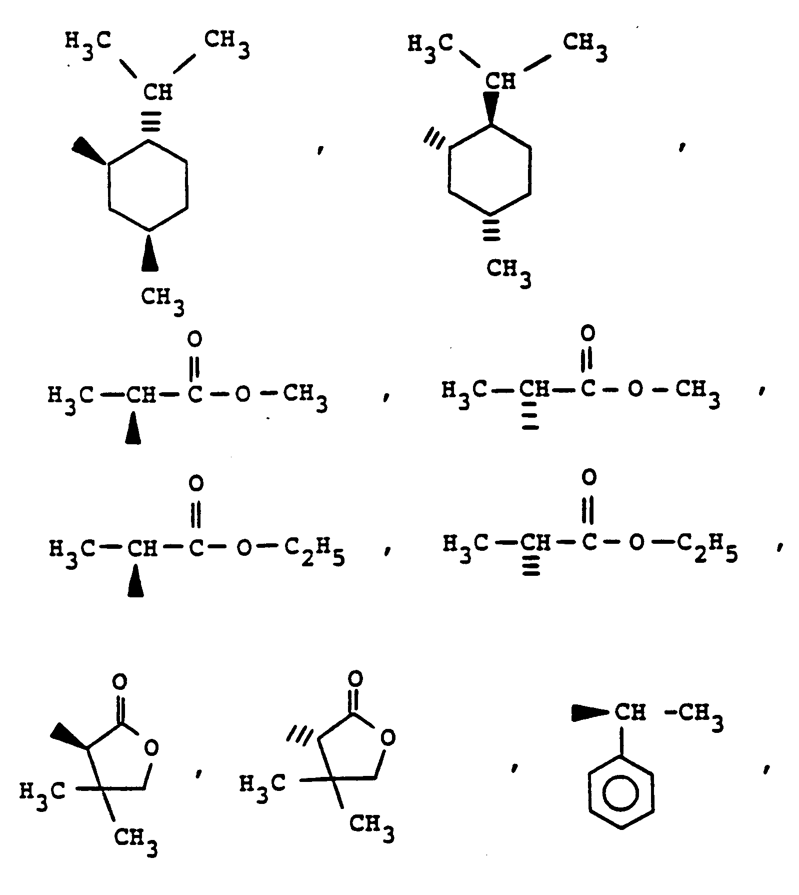

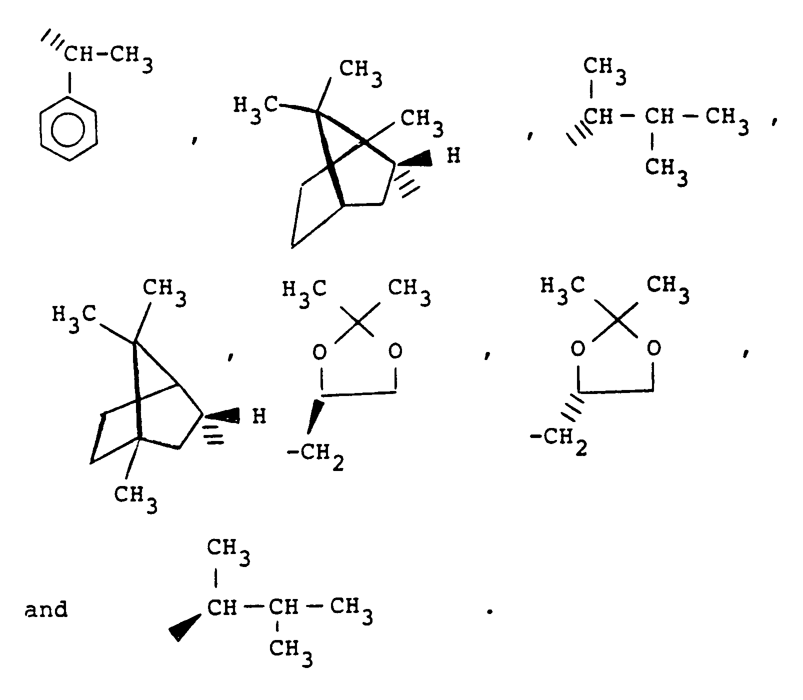

10. Use according to Claim 8 wherein R¹ is 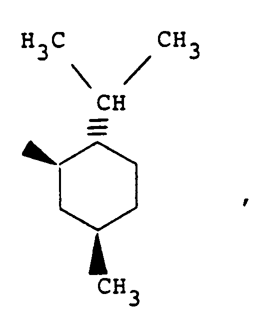 R² is methyl having the name, (1S-trans)-3,3-dimethoxy-1,2-cyclobutanedicarboxylic acid, di-(-)-menthyl ester.

## Patentansprüche (Patentansprüche für folgende(n) Vertragsstaat(en): AT, BE, CH, DE, DK, FR, GB, GR, IT, LI, LU, NL, SE,)

1. Verfahren zur Herstellung der optisch aktiven Verbindung 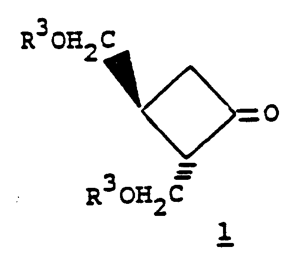 wobei R³ eine Schutzgruppe ist, das umfaßt:
(a) Umsetzen einer Verbindung der Formel 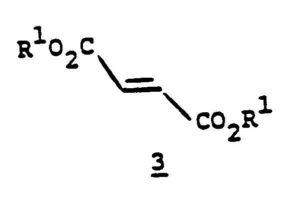 mit einer Verbindung der Formel 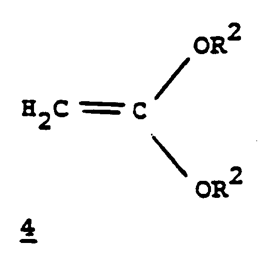 in Gegenwart einer Lewis-Säure, wobei ein Gemisch aus diastereomeren Isomeren der Formeln: 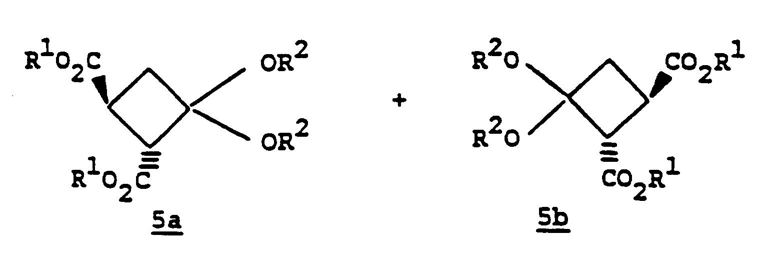 erhalten wird, wobei R¹ den Rest darstellt, der durch Entfernen der Hydroxygruppe aus dem homochiralen Alkohol der Formel R¹OH erhalten wird, und einen verzweigtkettigen Alkylrest mit 4 bis 20 Kohlenstoffatomen, einen substituierten gerad- oder verzweigtkettigen Alkylrest mit 1 bis 20 Kohlenstoffatomen, einen substituierten Cycloalkylrest mit 3 bis 20 Kohlenstoffatomen, einen verbrückten Cycloalkylrest mit 6 bis 20 Kohlenstoffatomen, einen substituierten verbrückten Cycloalkylrest mit 6 bis 20 Kohlenstoffatomen, einen Polycycloalkylrest mit 7 bis 20 Kohlenstoffatomen, einen substituierten Polycycloalkylrest mit 7 bis 20 Kohlenstoffatomen, wobei die Substituenten einer oder mehrere sind, die aus Niederalkylresten mit 1 bis 5 Kohlenstoffatomen, Halogenatomen, Niederalkoxyresten mit 1 bis 5 Kohlenstoffatomen, 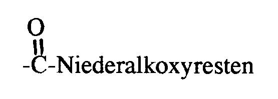 mit 1 bis 5 Kohlenstoffatomen, einer Gruppe 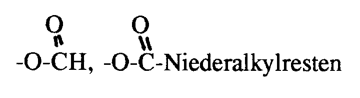 mit 1 bis 5 Kohlenstoffatomen und einer Phenylgruppe ausgewählt sind, einen Lactonrest aus 4 bis 6 Kohlenstoffatomen, einen substituierten Lactonrest aus 4 bis 6 Kohlenstoffatomen, wobei die Lactonsubstituenten einer oder mehrere sind, die aus Niederalkylresten mit 1 bis 5 Kohlenstoffatomen, Halogenatomen, Niederalkoxyresten mit 1 bis 5 Kohlenstoffatomen und einer Phenylgruppe ausgewählt sind, 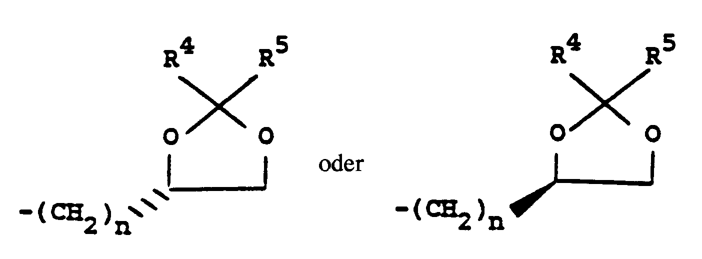 bedeutet, wobei n eine ganze Zahl von 1 bis 4 ist und R⁴ und R⁵ voneinander unabhängig aus einem Wasserstoffatom und Niederalkylresten mit 1 bis 5 Kohlenstoffatomen ausgewählt sind;
R² einen Niederalkylrest mit 1 bis 5 Kohlenstoffatomen darstellt oder beide Reste R² durch einen Alkylenrest mit 2 oder 3 Kohlenstoffatomen miteinander verbunden sind; mit der Maßgabe, daß die Lewis-Säure kein Titantetrachlorid, Zinntetrachlorid, Bortrifluoridetherat oder Bortribromid ist, wenn R¹ eine Gruppe 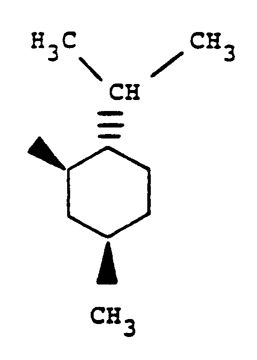 bedeutet und R² eine Methylgruppe darstellt;
(b) Erhalten der reinen Verbindung 5a durch Kristallisation oder Chromatographie;
(c) Umsetzen der Verbindung 5a mit einem Reduktionsmittel, wobei eine Verbindung der Formel 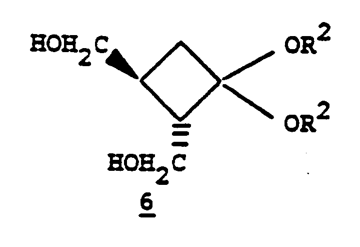 gebildet wird;
(d) Schützen der Hydroxygruppen der Verbindung 6 mit einem Rest (R³), wobei eine Verbindung der Formel 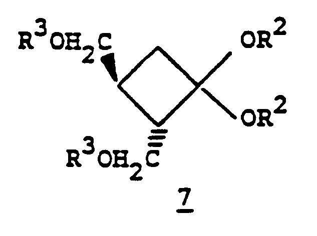 gebildet wird und
(e) Hydrolysieren einer Verbindung 7 unter Verwendung eines sauren Katalysators, wobei die Verbindung der Formel 1 gebildet wird.

2. Verfahren nach Anspruch 1, wobei die Lewis-Säure aus Diethylaluminiumchlorid, Diisobutylaluminiumchlorid, Ethylaluminiumdichlorid, Isopropoxyaluminiumdichlorid, Aluminiumtrichlorid, Bortrifluorid, Bortrichlorid, Titantetrachlorid, Dichlordiisopropoxytitan, Zinntetrachlorid und Zinntrichlorid ausgewählt wird.

3. Verfahren nach Anspruch 1, wobei die Lewis-Säure ein Di(niederalkyl)aluminiumchlorid ist, wobei jeder Niederalkylrest in der Lewis-Säure 2 bis 4 Kohlenstoffatome aufweist.

4. Verfahren nach Anspruch 1, 2 oder 3, wobei R¹ aus 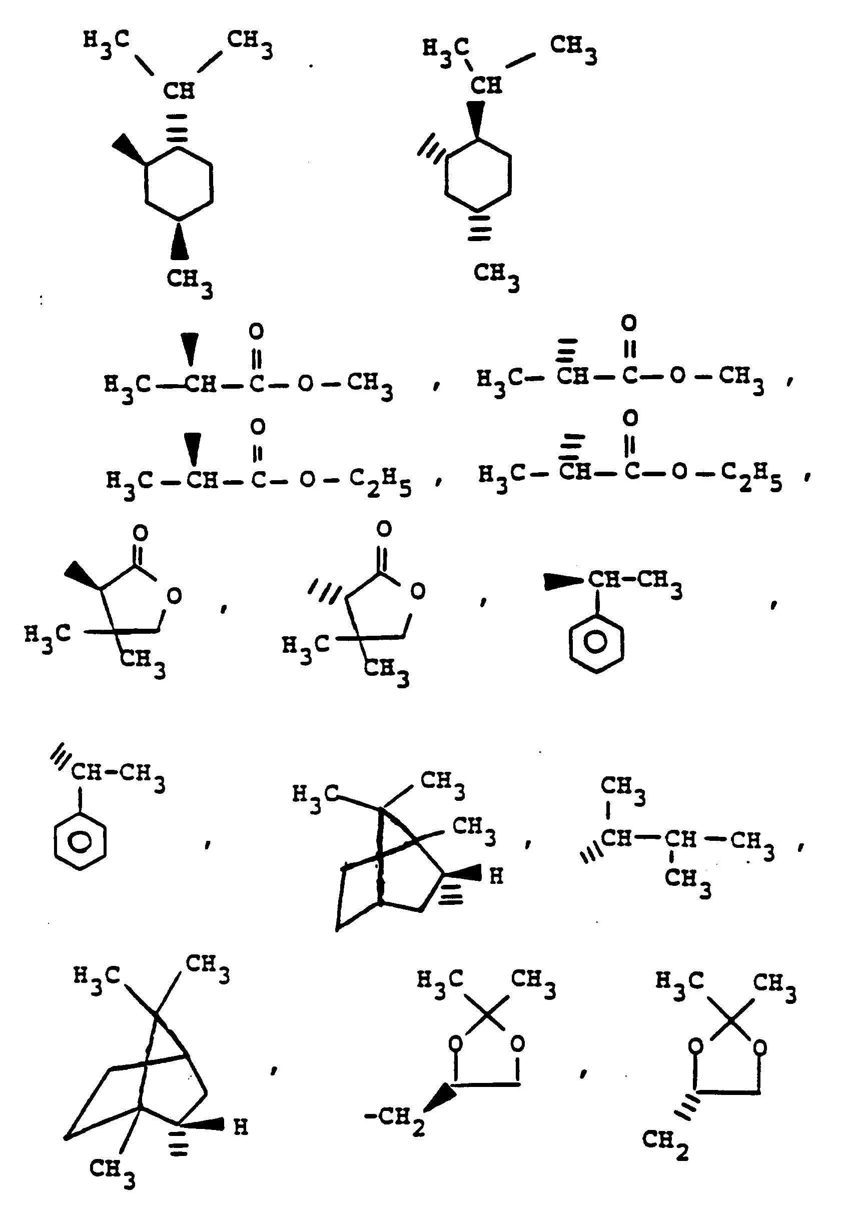 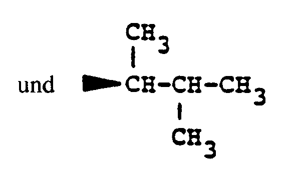 ausgewählt wird.

5. Verfahren nach Anspruch 1, 2 oder 3, wobei R¹ eine Gruppe 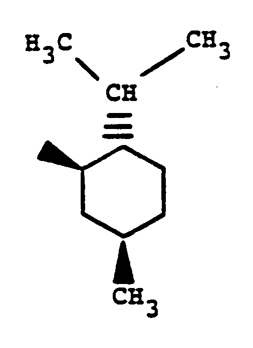 bedeutet und
R² eine Methylgruppe darstellt.

6. Verfahren nach einem der Ansprüche 1 - 5, wobei die Verbindung 4 in der Menge von 0,1 bis 5,0 Äquivalenten pro Äquivalent der Verbindung 3 vorliegt und die Lewis-Säure in einer Menge von 0,5 bis 5,0 Äquivalenten pro Äquivalent der Verbindung 3 vorliegt und wobei das Umsetzungsgemisch in Schritt (a) 1 Minute bis 24 Stunden bei einer Temperatur von -100°C bis 25°C gerührt wird.

7. Verfahren nach Anspruch 1, wobei R¹ eine Gruppe 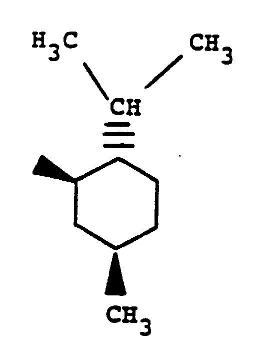 bedeutet,
R² eine Methylgruppe darstellt,
die Lewis-Säure Diisobutylaluminiumchlorid ist,
die Verbindung 4 in der Menge von 1,0 bis 2 Äquivalenten pro Äquivalent der Verbindung 3 vorliegt und die Lewis-Säure in einer Menge von 1,5 bis 2,5 Äquivalenten pro Äquivalent der Verbindung 3 vorliegt,
das Reduktionsmittel des Schritts (c) Lithiumaluminiumhydrid ist und die Schutzgruppe in Schritt (d) eine Benzoylgruppe ist.

8. Verbindung der Formel 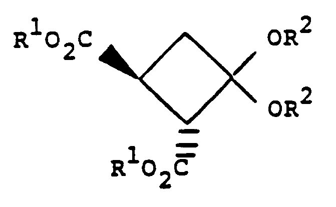 wobei die absolute Stereochemie am Cyclobutan (1S,2R) ist und R¹ den Rest darstellt, der durch Entfernen der Hydroxygruppe aus dem homochiralen Alkohol der Formel R¹OH erhalten wird, und einen verzweigtkettigen Alkylrest mit 4 bis 20 Kohlenstoffatomen, einen substituierten gerad- oder verzweigtkettigen Alkylrest mit 1 bis 20 Kohlenstoffatomen, einen substituierten Cycloalkylrest mit 3 bis 20 Kohlenstoffatomen, einen verbrückten Cycloalkylrest mit 6 bis 20 Kohlenstoffatomen, einen substituierten verbrückten Cycloalkylrest mit 6 bis 20 Kohlenstoffatomen, einen Polycycloalkylrest mit 7 bis 20 Kohlenstoffatomen, einen substituierten Polycycloalkylrest mit 7 bis 20 Kohlenstoffatomen, wobei die Substituenten einer oder mehrere sind, die aus Niederalkylresten mit 1 bis 5 Kohlenstoffatomen, Halogenatomen, Niederalkoxyresten mit 1 bis 5 Kohlenstoffatomen, 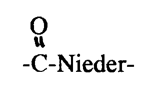 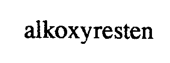 mit 1 bis 5 Kohlenstoffatomen, einer Gruppe 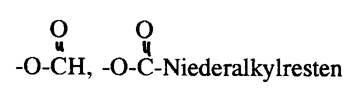 mit 1 bis 5 Kohlenstoffatomen und einer Phenylgruppe ausgewählt sind, einen Lactonrest aus 4 bis 6 Kohlenstoffatomen, einen substituierten Lactonrest aus 4 bis 6 Kohlenstoffatomen, wobei die Lactonsubstituenten einer oder mehrere sind, die aus Niederalkylresten mit 1 bis 5 Kohlenstoffatomen, Halogenatomen, Niederalkoxyresten mit 1 bis 5 Kohlenstoffatomen und einer Phenylgruppe ausgewählt sind, 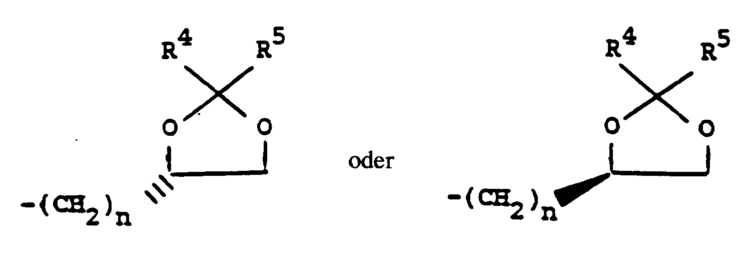 bedeutet, wobei n eine ganze Zahl von 1 bis 4 ist und R⁴ und R⁵ voneinander unabhängig aus einem Wasserstoffatom und Niederalkylresten mit 1 bis 5 Kohlenstoffatomen ausgewählt sind, und
R² einen Niederalkylrest mit 1 bis 5 Kohlenstoffatomen darstellt oder beide Reste R² durch einen Alkylenrest mit 2 oder 3 Kohlenstoffatomen miteinander verbunden sind.

9. Verbindung nach Anspruch 8, wobei R¹ aus 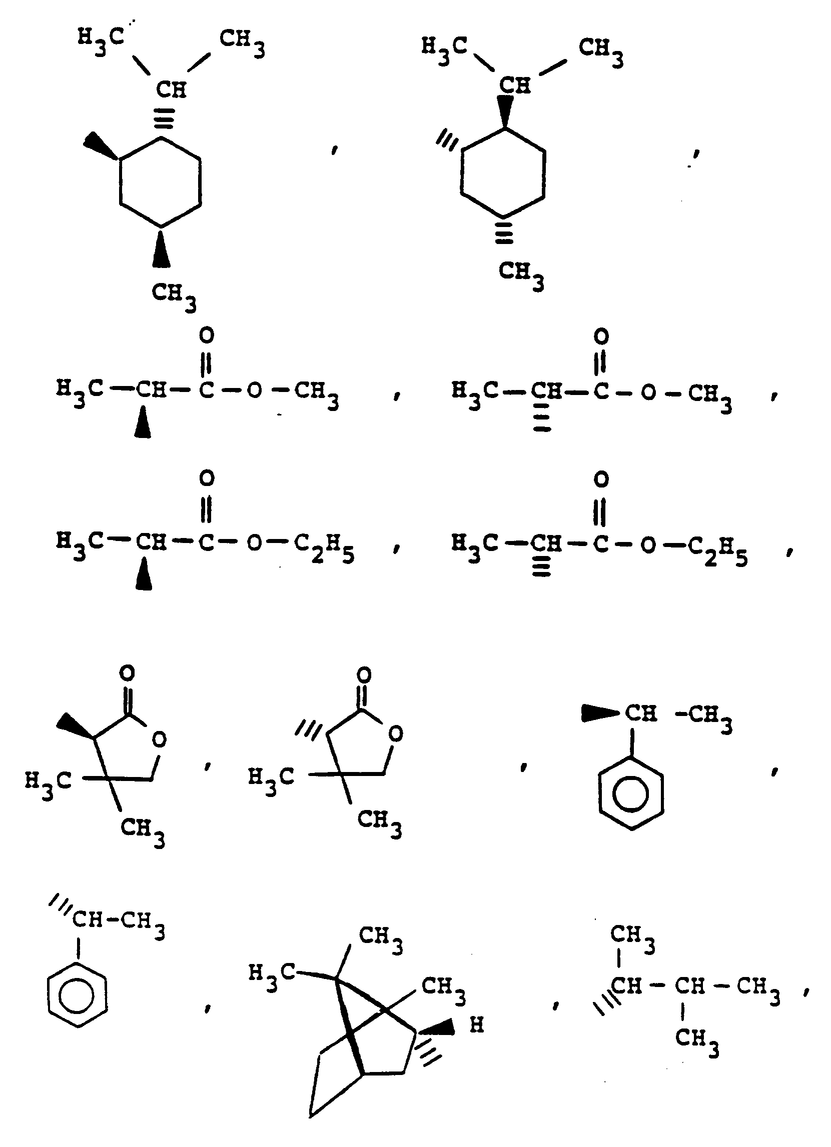 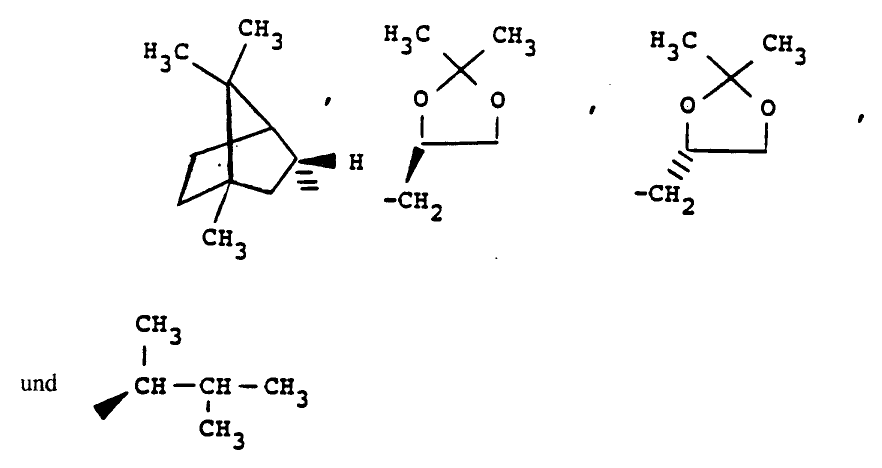 ausgewählt wird.

10. Verbindung nach Anspruch 8, wobei R¹ eine Gruppe 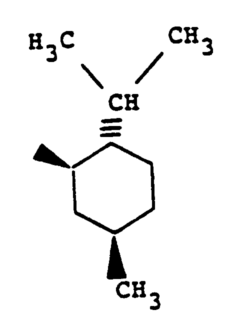 bedeutet und
R² eine Methylgruppe darstellt, mit Namen (1S-*trans*)-3,3-Dimethoxy-1,2-cyclobutandicarbonsäuredi-(-)-menthylester.

## Patentansprüche (Patentansprüche für folgende(n) Vertragsstaat(en): ES)

1. Verfahren zur Herstellung der optisch aktiven Verbindung 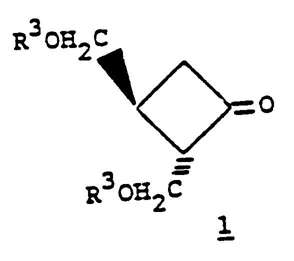 wobei R³ eine Schutzgruppe ist, das umfaßt:
(a) Umsetzen einer Verbindung der Formel 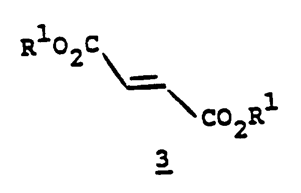 mit einer Verbindung der Formel 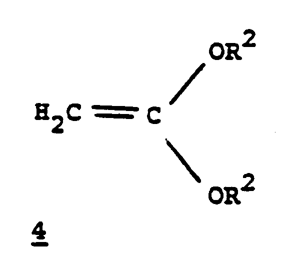 in Gegenwart einer Lewis-Säure, wobei ein Gemisch aus diastereomeren Isomeren der Formeln: 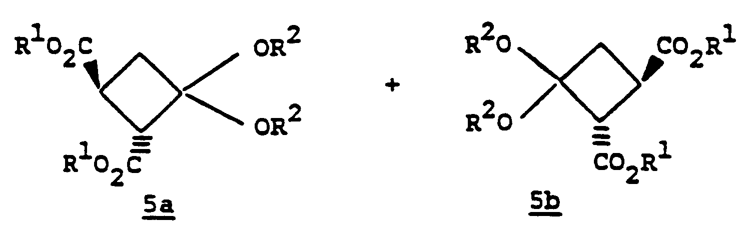 erhalten wird, wobei R¹ den Rest darstellt, der durch Entfernen der Hydroxygruppe aus dem homochiralen Alkohol der Formel R¹OH erhalten wird, und einen verzweigtkettigen Alkylrest mit 4 bis 20 Kohlenstoffatomen, einen substituierten gerad- oder verzweigtkettigen Alkylrest mit 1 bis 20 Kohlenstoffatomen, einen substituierten Cycloalkylrest mit 3 bis 20 Kohlenstoffatomen, einen verbrückten Cycloalkylrest mit 6 bis 20 Kohlenstoffatomen, einen substituierten verbrückten Cycloalkylrest mit 6 bis 20 Kohlenstoffatomen, einen Polycycloalkylrest mit 7 bis 20 Kohlenstoffatomen, einen substituierten Polycycloalkylrest mit 7 bis 20 Kohlenstoffatomen, wobei die Substituenten einer oder mehrere sind, die aus Niederalkylresten mit 1 bis 5 Kohlenstoffatomen, Halogenatomen, Niederalkoxyresten mit 1 bis 5 Kohlenstoffatomen, 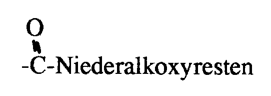 mit 1 bis 5 Kohlenstoffatomen, einer Gruppe 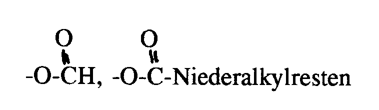 mit 1 bis 5 Kohlenstoffatomen und einer Phenylgruppe ausgewählt sind, einen Lactonrest aus 4 bis 6 Kohlenstoffatomen, einen substituierten Lactonrest aus 4 bis 6 Kohlenstoffatomen, wobei die Lactonsubstituenten einer oder mehrere sind, die aus Niederalkylresten mit 1 bis 5 Kohlenstoffatomen, Halogenatomen, Niederalkoxyresten mit 1 bis 5 Kohlenstoffatomen und einer Phenylgruppe ausgewählt sind, 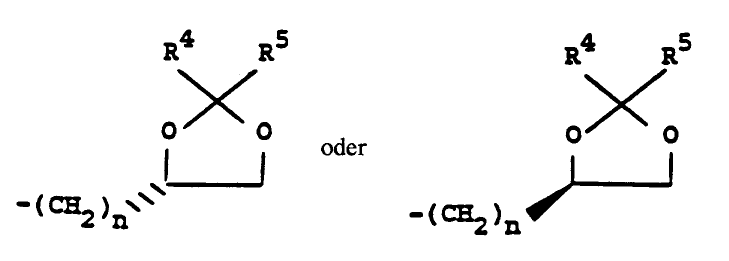 bedeutet, wobei n eine ganze Zahl von 1 bis 4 ist und R⁴ und R⁵ voneinander unabhängig aus einem Wasserstoffatom und Niederalkylresten mit 1 bis 5 Kohlenstoffatomen ausgewählt sind;
R² einen Niederalkylrest mit 1 bis 5 Kohlenstoffatomen darstellt oder beide Reste R² durch einen Alkylenrest mit 2 oder 3 Kohlenstoffatomen miteinander verbunden sind; mit der Maßgabe, daß die Lewis-Säure kein Titantetrachlorid, Zinntetrachlorid, Bortrifluoridetherat oder Bortribromid ist, wenn R¹ eine Gruppe 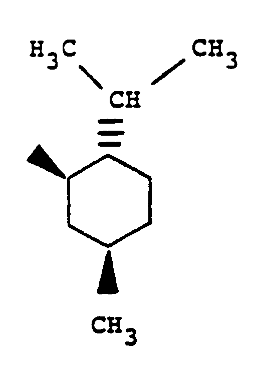
bedeutet und R² eine Methylgruppe darstellt;
(b) Erhalten der reinen Verbindung 5a durch Kristallisation oder Chromatographie;
(c) Umsetzen der Verbindung 5a mit einem Reduktionsmittel, wobei eine Verbindung der Formel 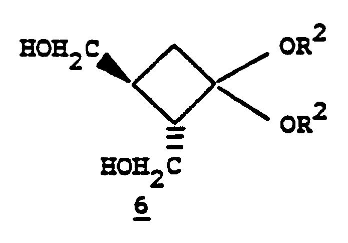 gebildet wird;
(d) Schützen der Hydroxygruppen der Verbindung 6 mit einem Rest (R³), wobei eine Verbindung der Formel 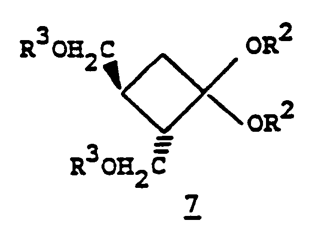 gebildet wird;
(e) Hydrolysieren einer Verbindung 7 unter Verwendung eines sauren Katalysators, wobei die Verbindung der Formel 1 gebildet wird.

2. Verfahren nach Anspruch 1, wobei die Lewis-Säure aus Diethylaluminiumchlorid, Diisobutylaluminiumchlorid, Ethylaluminiumdichlorid, Isopropoxyaluminiumdichlorid, Aluminiumtrichlorid, Bortrifluorid, Bortrichlorid, Titantetrachlorid, Dichlordiisopropoxytitan, Zinntetrachlorid und Zinntrichlorid ausgewählt wird.

3. Verfahren nach Anspruch 1, wobei die Lewis-Säure ein Di(niederalkyl)aluminiumchlorid ist, wobei jeder Niederalkylrest in der Lewis-Säure 2 bis 4 Kohlenstoffatome aufweist.

4. Verfahren nach Anspruch 1, wobei R¹ aus 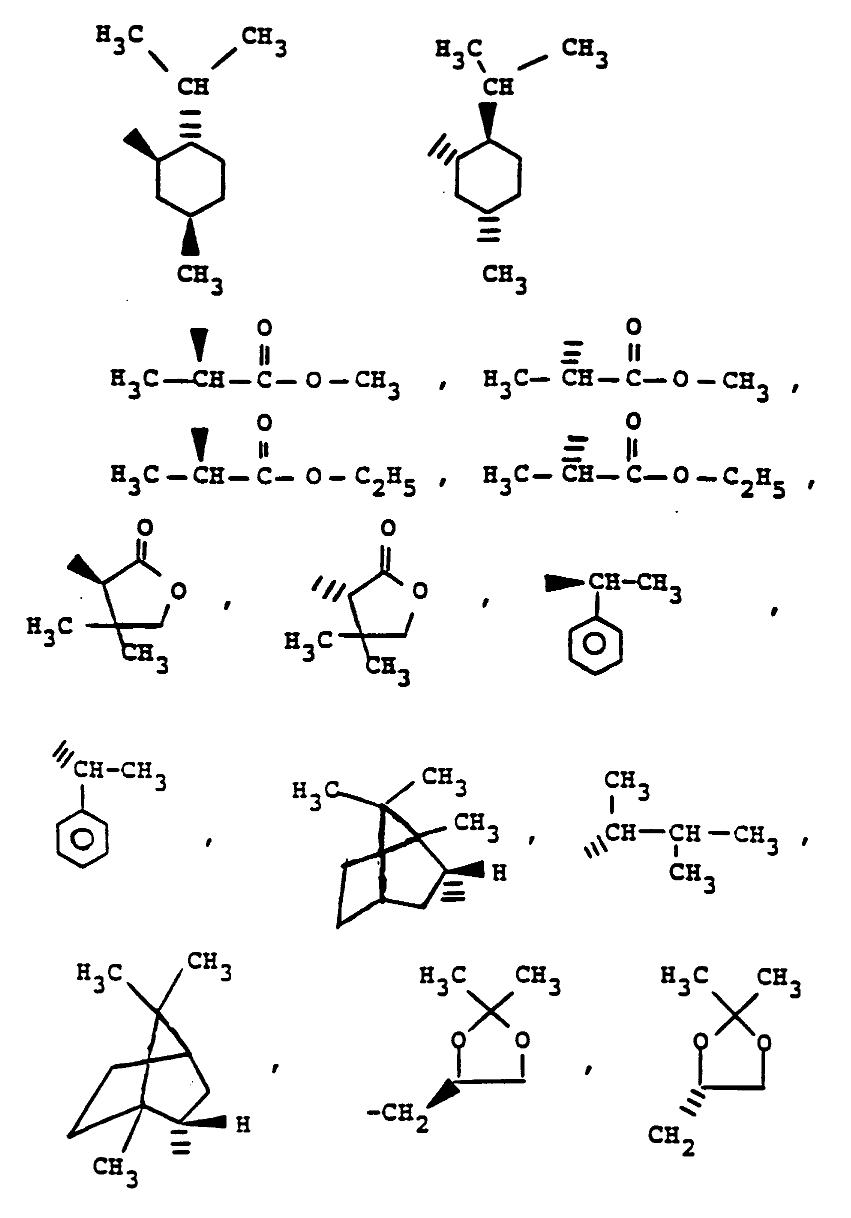 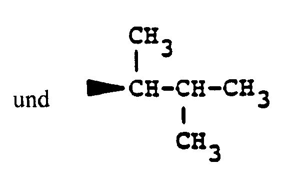 ausgewählt wird.

5. Verfahren nach Anspruch 1, wobei R¹ eine Gruppe 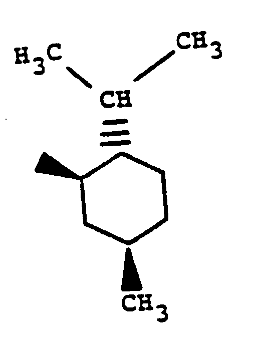 bedeutet und
R² eine Methylgruppe darstellt.

6. Verfahren nach Anspruch 1, wobei die Verbindung 4 in der Menge von 0,1 bis 5,0 Äquivalenten pro Äquivalent der Verbindung 3 vorliegt und die Lewis-Säure in einer Menge von 0,5 bis 5,0 Äquivalenten pro Äquivalent der Verbindung 3 vorliegt und wobei das Umsetzungsgemisch in Schritt (a) 1 Minute bis 24 Stunden bei einer Temperatur von -100°C bis 25°C gerührt wird.

7. Verfahren nach Anspruch 1, wobei R¹ eine Gruppe 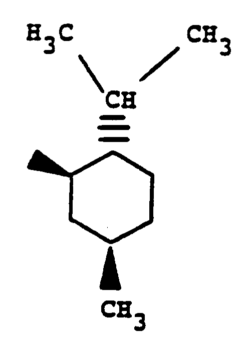 bedeutet,
R² eine Methylgruppe darstellt,
die Lewis-Säure Diisobutylaluminiumchlorid ist,
Verbindung 4 in der Menge von 1,0 bis 2 Äquivalenten pro Äquivalent der Verbindung 3 vorliegt und die Lewis-Säure in einer Menge von 1,5 bis 2,5 Äquivalenten pro Äquivalent der Verbindung 3 vorliegt,
das Reduktionsmittel des Schritts (c) Lithiumaluminiumhydrid ist und
die Schutzgruppe in Schritt (d) eine Benzoylgruppe ist.

8. Verwendung einer Verbindung der Formel 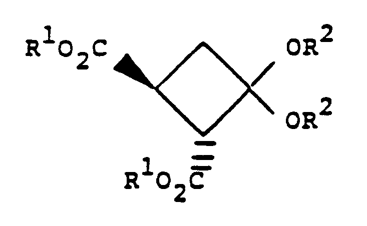 wobei die absolute Stereochemie am Cyclobutan (1S,2R) ist und R¹ den Rest darstellt, der durch Entfernen der Hydroxygruppe aus dem homochiralen Alkohol der Formel R¹OH erhalten wird, und einen verzweigtkettigen Alkylrest mit 4 bis 20 Kohlenstoffatomen, einen substituierten gerad- oder verzweigtkettigen Alkylrest mit 1 bis 20 Kohlenstoffatomen, einen substituierten Cycloalkylrest mit 3 bis 20 Kohlenstoffatomen, einen verbrückten Cycloalkylrest mit 6 bis 20 Kohlenstoffatomen, einen substituierten verbrückten Cycloalkylrest mit 6 bis 20 Kohlenstoffatomen, einen Polycycloalkylrest mit 7 bis 20 Kohlenstoffatomen, einen substituierten Polycycloalkylrest mit 7 bis 20 Kohlenstoffatomen, wobei die Substituenten einer oder mehrere sind, die aus Niederalkylresten mit 1 bis 5 Kohlenstoffatomen, Halogenatomen, Niederalkoxyresten mit 1 bis 5 Kohlenstoffatomen, 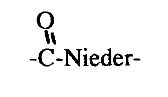 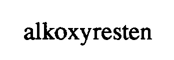 mit 1 bis 5 Kohlenstoffatomen, einer Gruppe 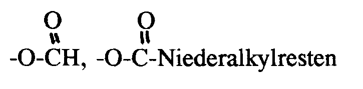 mit 1 bis 5 Kohlenstoffatomen und einer Phenylgruppe ausgewählt sind, einen Lactonrest aus 4 bis 6 Kohlenstoffatomen, einen substituierten Lactonrest aus 4 bis 6 Kohlenstoffatomen, wobei die Lactonsubstituenten einer oder mehrere sind, die aus Niederalkylresten mit 1 bis 5 Kohlenstoffatomen, Halogenatomen, Niederalkoxyresten mit 1 bis 5 Kohlenstoffatomen und einer Phenylgruppe ausgewählt sind, 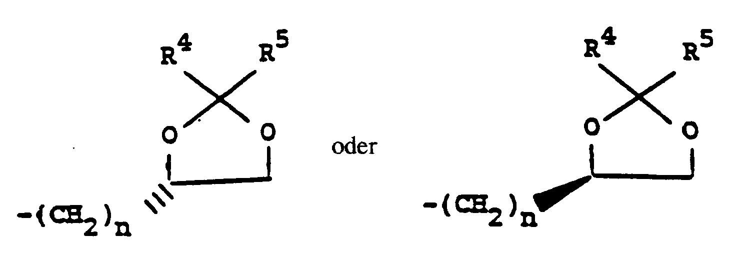 bedeutet, wobei n eine ganze Zahl von 1 bis 4 ist und R⁴ und R⁵ voneinander unabhängig aus einem Wasserstoffatom und Niederalkylresten mit 1 bis 5 Kohlenstoffatomen ausgewählt sind, und
R² einen Niederalkylrest mit 1 bis 5 Kohlenstoffatomen darstellt oder beide Reste R² durch einen Alkylenrest mit 2 oder 3 Kohlenstoffatomen miteinander verbunden sind, zur Herstellung einer Verbindung der Formel 1, wie in Anspruch 1 definiert.

9. Verwendung nach Anspruch 8, wobei R¹ aus 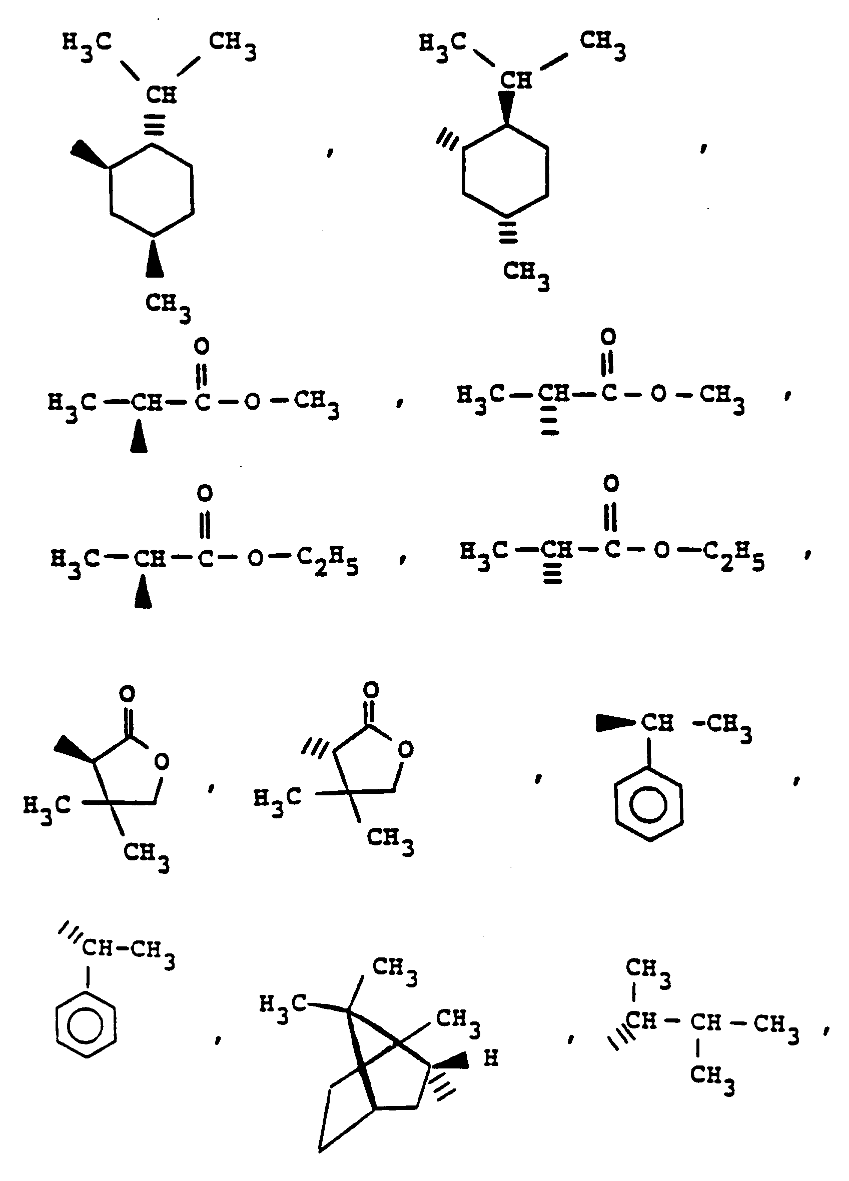 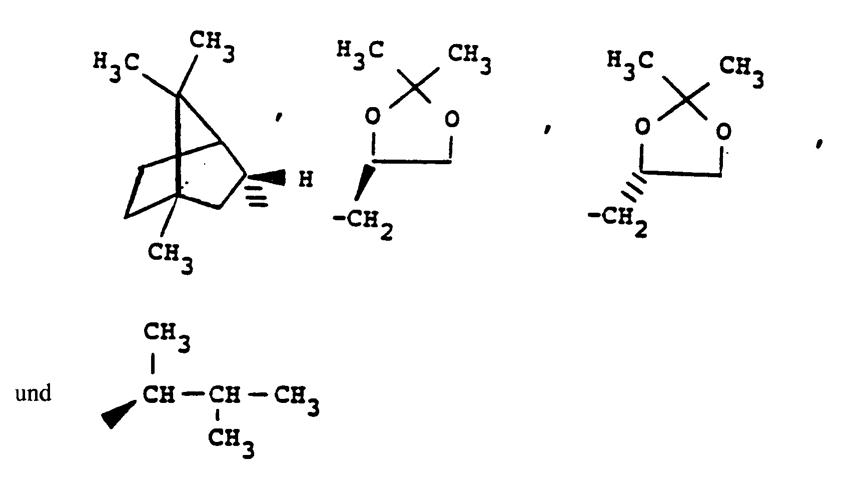 ausgewählt wird.

10. Verwendung nach Anspruch 8, wobei R¹ eine Gruppe 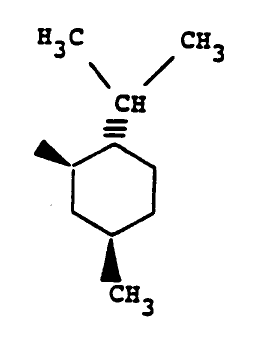 bedeutet und
R² eine Methylgruppe darstellt, mit Namen (1S-*trans*)-3,3-Dimethoxy-1,2-cyclobutandicarbonsäuredi-(-)-menthylester.

## Revendications (Revendications pour l'(les) Etat(s) contractant(s) suivant(s): AT, BE, CH, DE, DK, FR, GB, GR, IT, LI, LU, NL, SE,)

1. Procédé de préparation du composé optiquement actif de formule 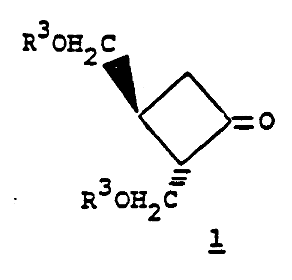 dans laquelle R³ est un groupe protecteur, qui consiste:
(a) à faire réagir un composé de formule 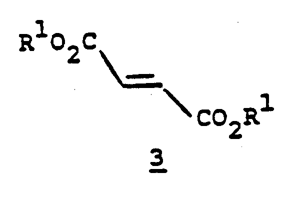 avec un composé de formule 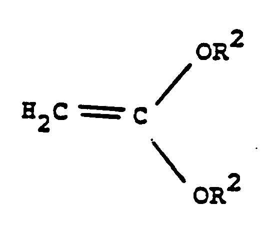 en présence d'un acide de Lewis, pour obtenir un mélange de composés diastéréoisomères de formules: 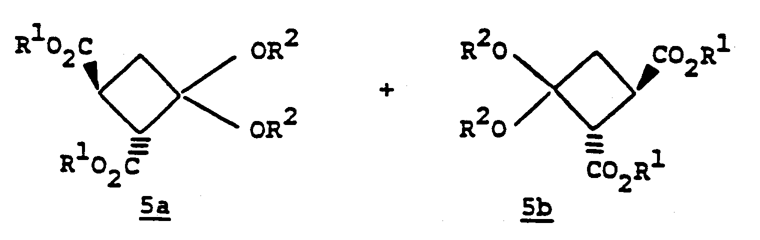 dans lesquelles R¹ est le groupe obtenu par élimination du groupe hydroxy de l'alcool homochiral de formule R¹OH et est un groupe alkyle à chaîne ramifiée de 4 à 20 atomes de carbone, un groupe alkyle substitué à chaîne droite ou ramifiée de 1 à 20 atomes de carbone, un groupe cycloalkyle substitué de 3 à 20 atomes de carbone, un groupe cycloalkyle ponté de 6 à 20 atomes de carbone, un groupe cycloalkyle ponté et substitué de 6 à 20 atomes de carbone, un groupe polycycloalkyle de 7 à 20 atomes de carbone, un groupe polycycloalkyle substitué de 7 à 20 atomes de carbone, le nombre desdits substituants étant de un ou plus et lesdits substituants étant choisis entre les groupes alkyle inférieur de 1 à 5 atomes de carbone, les atomes d'halogène, les groupes alcoxy inférieur de 1 à 5 atomes de carbone, 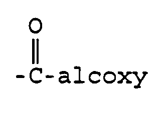 inférieur de 1 à 5 atomes de carbone, 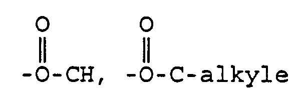 inférieur de 1 à 5 atomes de carbone et le groupe phényle, une lactone de 4 à 6 atomes de carbone, une lactone substituée de 4 à 6 atomes de carbone, le nombre desdits substituants de la lactone étant de un ou plus et lesdits substituants étant choisis entre les groupes alkyle inférieur de 1 à 5 atomes de carbone, les atomes d'halogène, les groupes alcoxy inférieur de 1 à 5 atomes de carbone, et le groupe phényle, 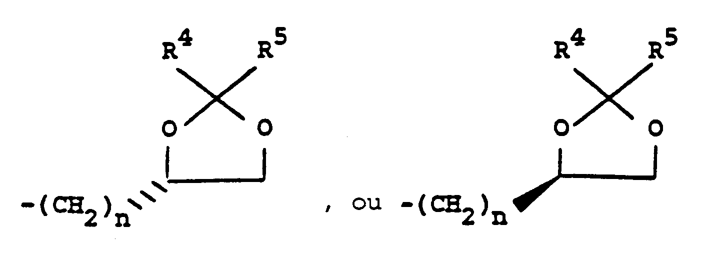 où n est un nombre entier de 1 à 4, et R⁴ et R⁵ sont choisis indépendamment entre l'hydrogène et les groupes alkyle inférieur de 1 à 5 atomes de carbone;
R² est un groupe alkyle inférieur de 1 à 5 atomes de carbone ou bien les deux groupes R² sont réunis par un groupe alkylène de 2 ou 3 atomes de carbone; sous réserve que, lorsque R¹ est 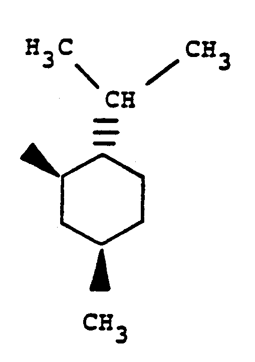 et R² est le groupe méthyle, alors l'acide de Lewis ne peut être le tétrachlorure de titane, le tétrachlorure d'étain, un éthérat de trifluorure de bore ou le tribromure de bore;
(b) à obtenir le composé pur 5a par cristallisation ou chromatographie;
(c) à faire réagir le composé 5a avec un réducteur pour former un composé de formule 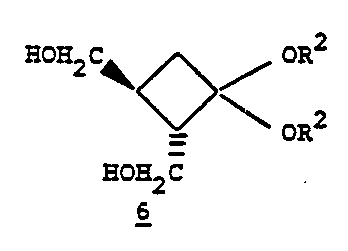
(d) à protéger les groupes hydroxy du composé 6 par un groupe (R³) pour former un composé de formule 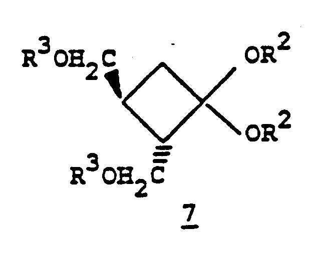 et
(e) à hydrolyser le composé 7 à l'aide d'un catalyseur qui est un acide pour former le composé de formule 1.

2. Procédé selon la revendication 1, dans lequel l'acide de Lewis est choisi entre le chlorure de diéthylaluminium, le chlorure de diisobutylaluminium, le dichlorure d'éthylaluminium, le dichlorure d'isopropoxyaluminium, le trichlorure d'aluminium, le trichlorure de bore, le trichlorure de bore, le tétrachlorure de titane, le dichlorodiisopropoxytitane, le tétrachlorure d'étain et le trichlorure d'étain.

3. Procédé selon la revendication 1, dans lequel l'acide de Lewis est un chlorure de di(alkyl inférieur) aluminium, chaque groupe alkyle inférieur de l'acide de Lewis ayant de 2 à 4 atomes de carbone.

4. Procédé selon la revendication 1, 2 ou 3, dans lequel R¹ est choisi entre

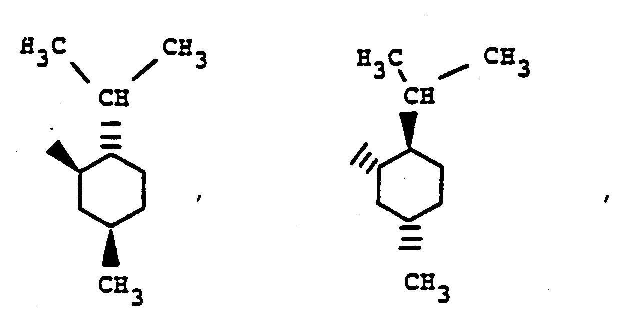

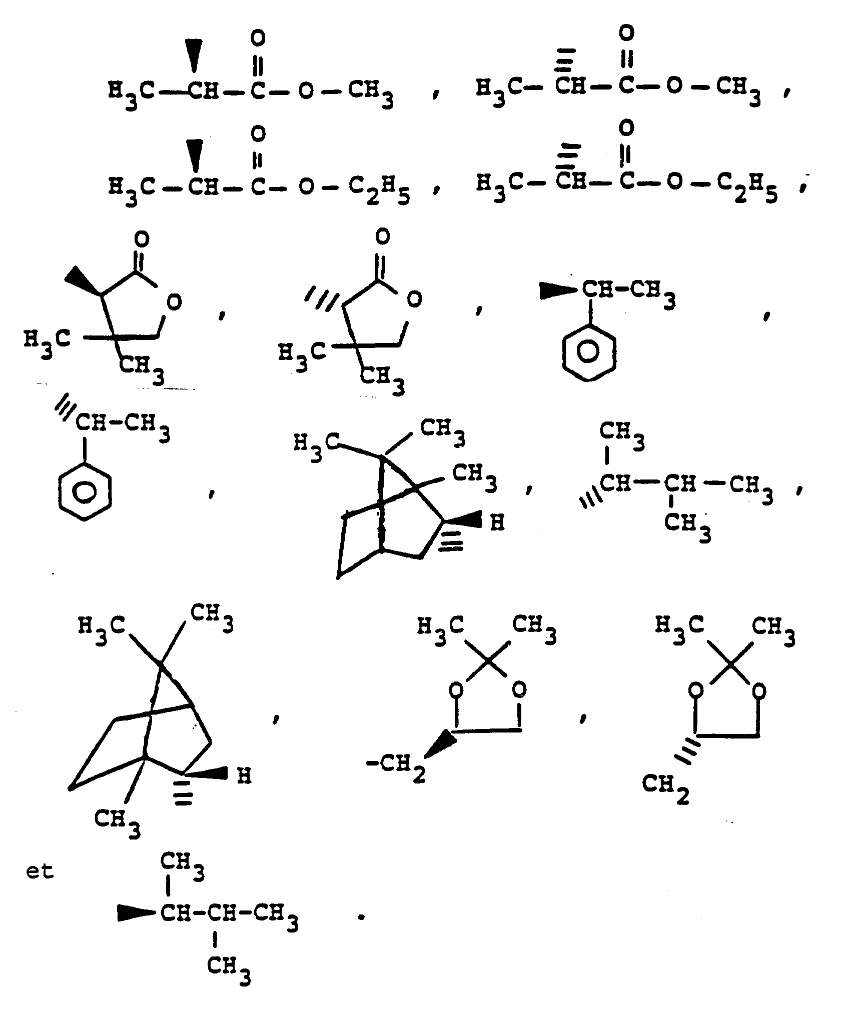

5. Procédé selon la revendication 1, 2 ou 3, dans lequel R¹ est 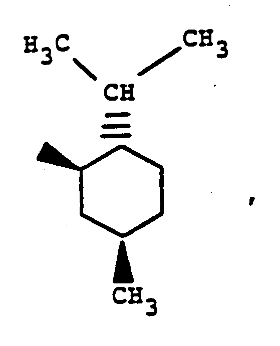 et
R² est le groupe méthyle.

6. Procédé selon l'une quelconque des revendications 1 à 5, dans lequel le composé 4 est présent en quantité de 0,1 à 5,0 équivalents par équivalent du composé 3 et l'acide de Lewis est présent en quantité de 0,5 à 5,0 équivalents par équivalent du composé 3, et dans lequel on agite le mélange réactionnel, dans l'étape (a), pendant 1 minute à 24 heures à une température de -100°C à +25°C.

7. Procédé selon la revendication 1, dans lequel R¹ est 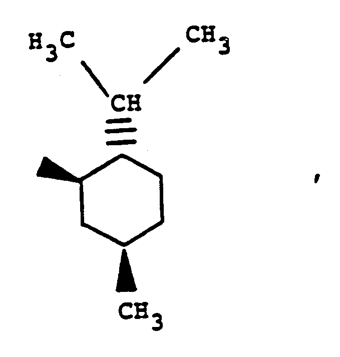 R² est le groupe méthyle,
l'acide de Lewis est le chlorure de diisobutylaluminium,
le composé 4 est présent en quantité de 1,0 à 2 équivalents par équivalent du composé 3 et l'acide de Lewis est présent en quantité de 1,5 à 2,5 équivalents par équivalent du composé 3,
le réducteur de l'étape (c) est l'hydrure de lithium et d'aluminium, et
le groupe protecteur, dans l'étape (d), est le groupe benzoyle.

8. Composé de formule 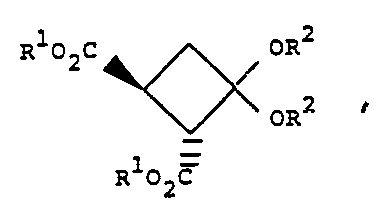 dans laquelle la stéréochimie absolue du cyclobutane est (1S, 2R) et R¹ est le groupe obtenu par élimination du groupe hydroxy de l'alcool homochiral de formule R¹OH et est un groupe alkyle à chaîne ramifiée de 4 à 20 atomes de carbone, un groupe alkyle substitué à chaîne droite ou ramifiée de 1 à 20 atomes de carbone, un groupe cycloalkyle substitué de 3 à 20 atomes de carbone, un groupe cycloalkyle ponté de 6 à 20 atomes de carbone, un groupe cycloalkyle ponté et substitué de 6 à 20 atomes de carbone, un groupe polycycloalkyle de 7 à 20 atomes de carbone, un groupe polycycloalkyle substitué de 7 à 20 atomes de carbone, lesdits substituants étant au nombre de un ou plus et étant choisis entre les groupes alkyle inférieur de 1 à 5 atomes de carbone, les atomes d'halogène, les groupes alcoxy inférieur de 1 à 5 atomes de carbone, 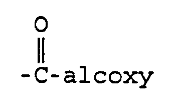 inférieur de 1 à 5 atomes de carbone, 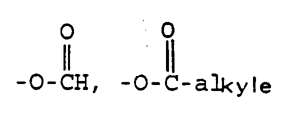 inférieur de 1 à 5 atomes de carbone, et le groupe phényle, une lactone de 4 à 6 atomes de carbone, une lactone substituée de 4 à 6 atomes de carbone, lesdits substituants de la lactone étant au nombre de un ou plus et étant choisis entre les groupes alkyle inférieur de 1 à 5 atomes de carbone, les atomes d'halogène, les groupes alcoxy inférieur de 1 à 5 atomes de carbone, et le groupe phényle, 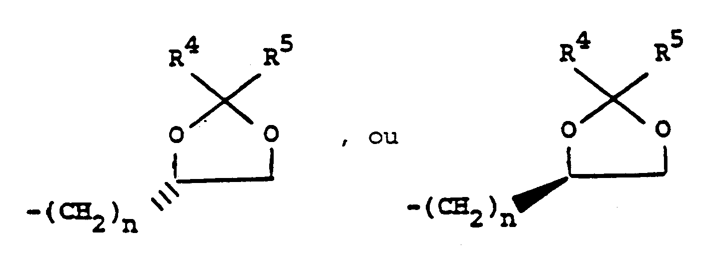 où n est un nombre entier de 1 à 4 et R⁴ et R⁵ sont choisis indépendamment entre l'hydrogène et les groupes alkyle inférieur de 1 à 5 atomes de carbone; et
R² est un groupe alkyle inférieur de 1 à 5 atomes de carbone, ou bien les deux groupes R² sont réunis par un groupe alkylène de 2 ou 3 atomes de carbone.

9. Composé selon la revendication 8, dans lequel R¹ est choisi entre

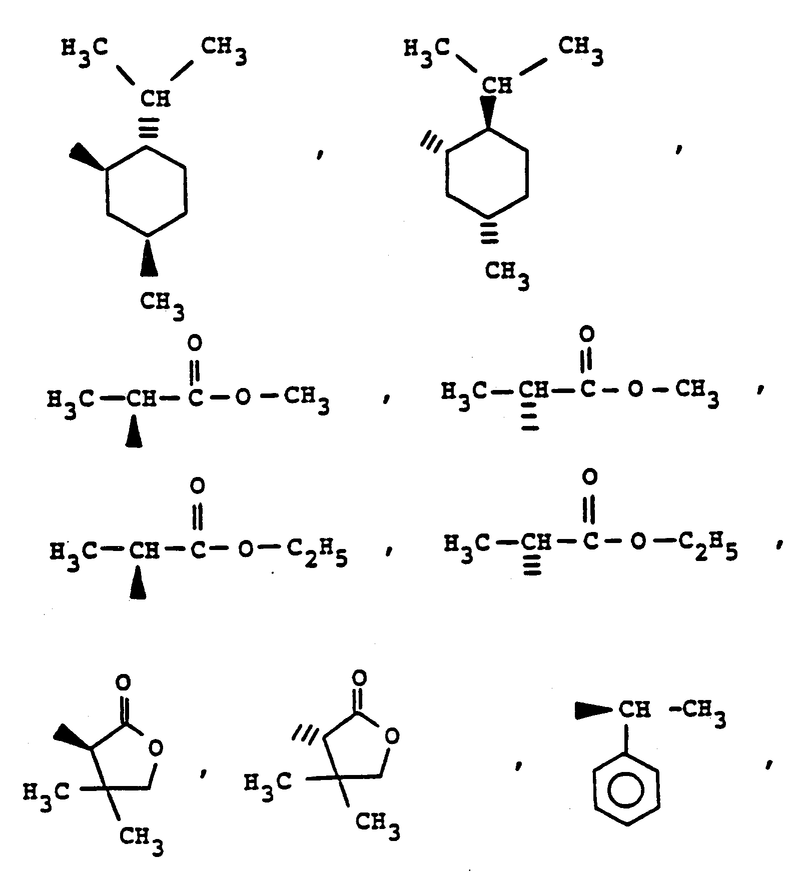

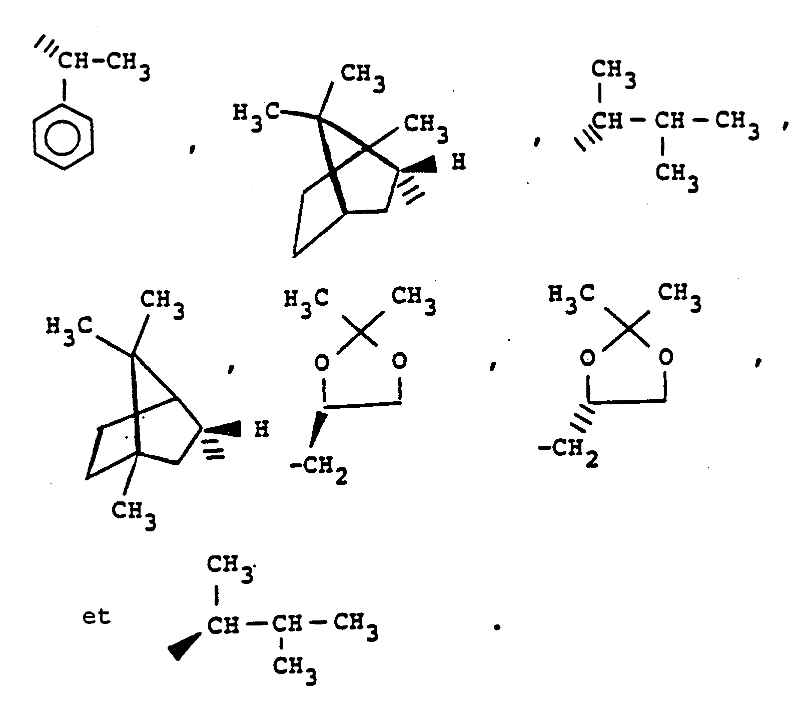

10. Composé selon la revendication 8, dans lequel R¹ est 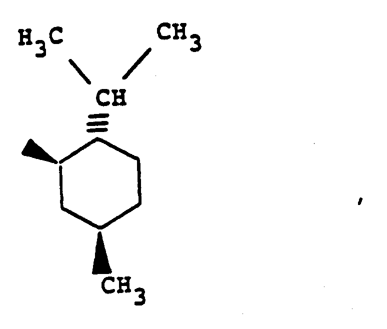 et
R² est le groupe méthyle, dont le nom est ester di-(-)-mentylique de l'acide (1S-trans)-3,3-diméthoxy-1,2-cyclobutanedicarboxylique.

## Revendications (Revendications pour l'(les) Etat(s) contractant(s) suivant(s): ES)

1. Procédé de préparation du composé optiquement actif de formule 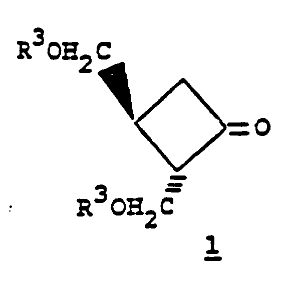 dans laquelle R³ est un groupe protecteur, qui consiste:
(a) à faire réagir un composé de formule 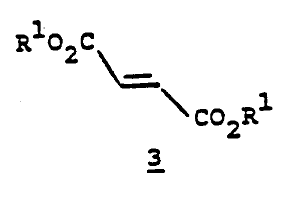 avec un composé de formule 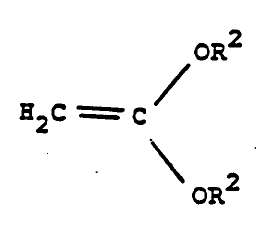 en présence d'un acide de Lewis, pour obtenir un mélange de composés diastéréoisomères de formules: 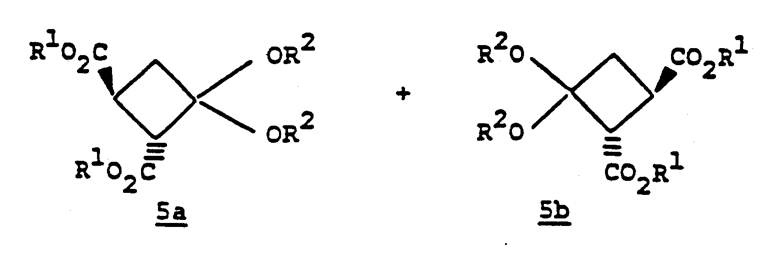 dans lesquelles R¹ est le groupe obtenu par élimination du groupe hydroxy de l'alcool homochiral de formule R¹OH et est un groupe alkyle à chaîne ramifiée de 4 à 20 atomes de carbone, un groupe alkyle substitué à chaîne droite ou ramifiée de 1 à 20 atomes de carbone, un groupe cycloalkyle substitué de 3 à 20 atomes de carbone, un groupe cycloalkyle ponté de 6 à 20 atomes de carbone, un groupe cycloalkyle ponté et substitué de 6 à 20 atomes de carbone, un groupe polycycloalkyle de 7 à 20 atomes de carbone, un groupe polycycloalkyle substitué de 7 à 20 atomes de carbone, le nombre desdits substituants étant de un ou plus et lesdits substituants étant choisis entre les groupes alkyle inférieur de 1 à 5 atomes de carbone, les atomes d'halogène, les groupes alcoxy inférieur de 1 à 5 atomes de carbone, 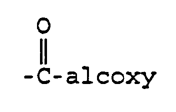 inférieur de 1 à 5 atomes de carbone, 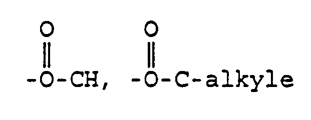 inférieur de 1 à 5 atomes de carbone et le groupe phényle, une lactone de 4 à 6 atomes de carbone, une lactone substituée de 4 à 6 atomes de carbone, le nombre desdits substituants de la lactone étant de un ou plus et lesdits substituants étant choisis entre les groupes alkyle inférieur de 1 à 5 atomes de carbone, les atomes d'halogène, les groupes alcoxy inférieur de 1 à 5 atomes de carbone, et le groupe phényle, 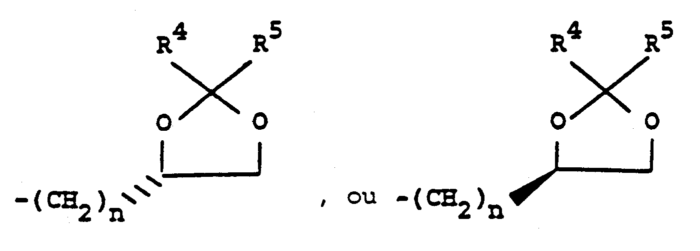 où n est un nombre entier de 1 à 4, et R⁴ et R⁵ sont choisis indépendamment entre l'hydrogène et les groupes alkyle inférieur de 1 à 5 atomes de carbone;
R² est un groupe alkyle inférieur de 1 à 5 atomes de carbone ou bien les deux groupes R² sont réunis par un groupe alkylène de 2 ou 3 atomes de carbone; sous réserve que, lorsque R¹ est 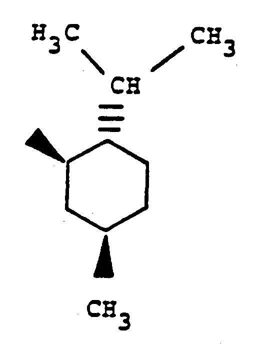 et R² est le groupe méthyle, alors l'acide de Lewis ne peut être le tétrachlorure de titane, le tétrachlorure d'étain, un éthérat de trifluorure de bore ou le tribromure de bore;
(b) à obtenir le composé pur 5a par cristallisation ou chromatographie;
(c) à faire réagir le composé 5a avec un réducteur pour former un composé de formule 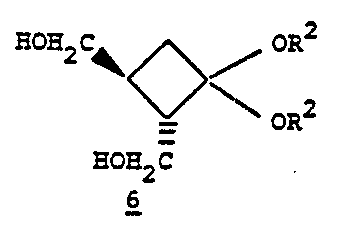
(d) à protéger les groupes hydroxy du composé 6 par un groupe (R³) pour former un composé de formule 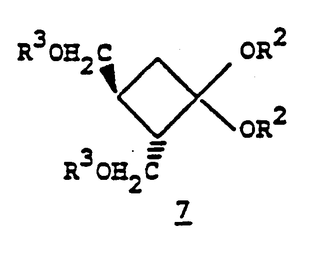 et
(e) à hydrolyser le composé 7 à l'aide d'un catalyseur qui est un acide pour former le composé de formule 1.

2. Procédé selon la revendication 1, dans lequel l'acide de Lewis est choisi entre le chlorure de diéthylaluminium, le chlorure de diisobutylaluminium, le dichlorure d'éthylaluminium, le dichlorure d'isopropoxyaluminium, le trichlorure d'aluminium, le trichlorure de bore, le trichlorure de bore, le tétrachlorure de titane, le dichlorodiisopropoxytitane, le tétrachlorure d'étain et le trichlorure d'étain.

3. Procédé selon la revendication 1, dans lequel l'acide de Lewis est un chlorure de di(alkyl inférieur) aluminium, chaque groupe alkyle inférieur de l'acide de Lewis ayant de 2 à 4 atomes de carbone.

4. Procédé selon la revendication 1, dans lequel R¹ est choisi entre

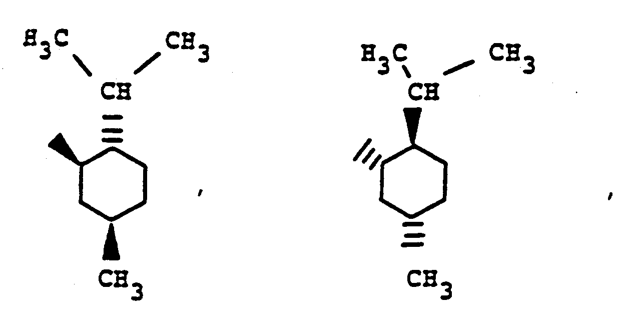

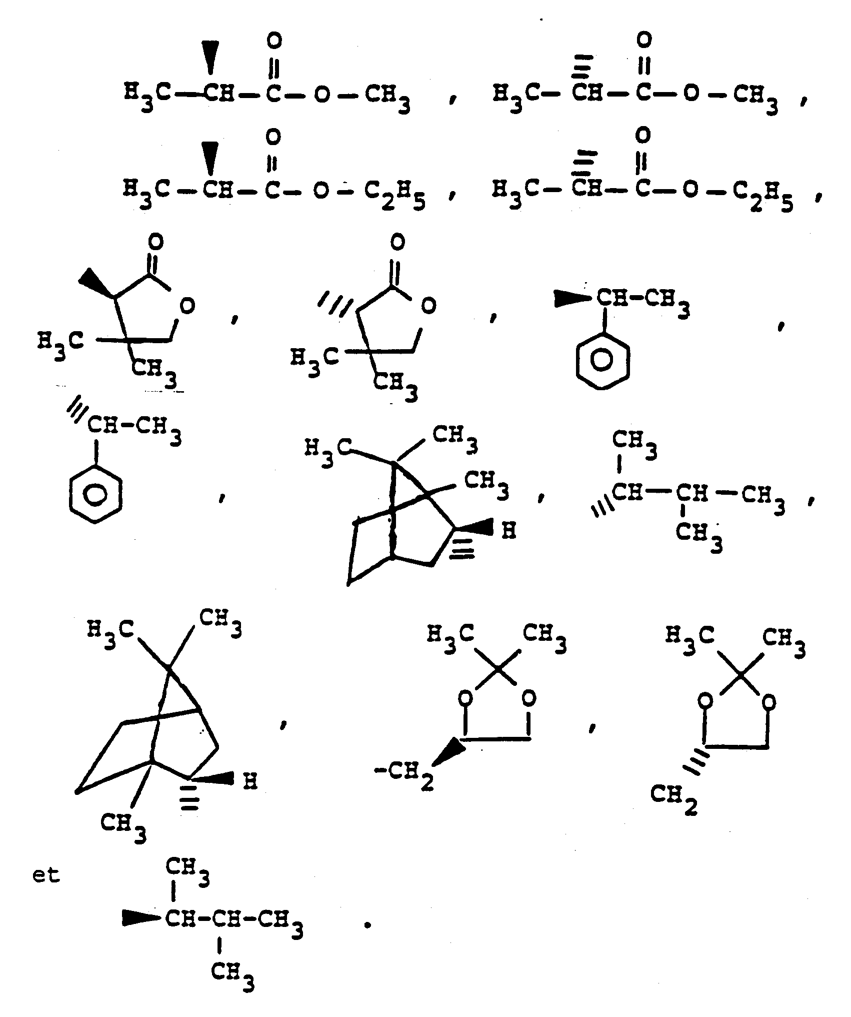

5. Procédé selon la revendication 1, dans lequel R¹ est 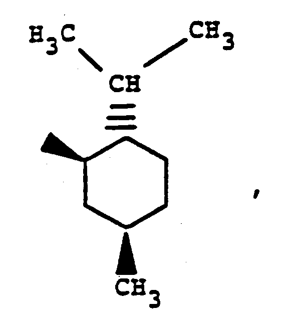 et R² est le groupe méthyle.

6. Procédé selon la revendication 1 , dans lequel le composé 4 est présent en quantité de 0,1 à 5,0 équivalents par équivalent du composé 3 et l'acide de Lewis est présent en quantité de 0,5 à 5,0 équivalents par équivalent du composé 3, et dans lequel on agite le mélange réactionnel, dans l'étape (a), pendant 1 minute à 24 heures à une température de -100°C à +25°C.

7. Procédé selon la revendication 1, dans lequel R¹ est 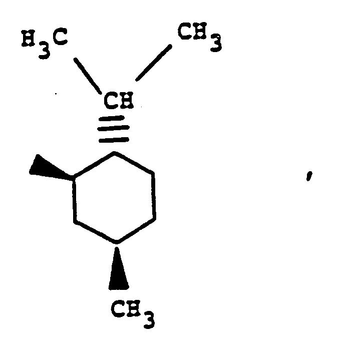 R² est le groupe méthyle,
l'acide de Lewis est le chlorure de diisobutylaluminium,
le composé 4 est présent en quantité de 1,0 à 2 équivalents par équivalent du composé 3 et l'acide de Lewis est présent en quantité de 1,5 à 2,5 équivalents par équivalent du composé 3,
le réducteur de l'étape (c) est l'hydrure de lithium et d'aluminium, et
le groupe protecteur, dans l'étape (d), est le groupe benzoyle.

8. Utilisation d'un composé de formule 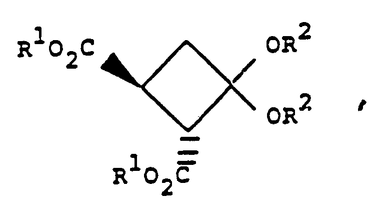 dans laquelle la stéréochimie absolue du cyclobutane est (1S, 2R) et R¹ est le groupe obtenu par élimination du groupe hydroxy de l'alcool homochiral de formule R¹OH et est un groupe alkyle à chaîne ramifiée de 4 à 20 atomes de carbone, un groupe alkyle substitué à chaîne droite ou ramifiée de 1 à 20 atomes de carbone, un groupe cycloalkyle substitué de 3 à 20 atomes de carbone, un groupe cycloalkyle ponté de 6 à 20 atomes de carbone, un groupe cycloalkyle ponté et substitué de 6 à 20 atomes de carbone, un groupe polycycloalkyle de 7 à 20 atomes de carbone, un groupe polycycloalkyle substitué de 7 à 20 atomes de carbone, lesdits substituants étant au nombre de un ou plus et étant choisis entre les groupes alkyle inférieur de 1 à 5 atomes de carbone, les atomes d'halogène, les groupes alcoxy inférieur de 1 à 5 atomes de carbone, 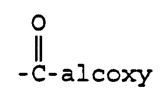 inférieur de 1 à 5 atomes de carbone, 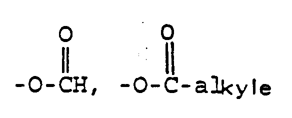 inférieur de 1 à 5 atomes de carbone, et le groupe phényle, une lactone de 4 à 6 atomes de carbone, une lactone substituée de 4 à 6 atomes de carbone, lesdits substituants de la lactone étant au nombre de un ou plus et étant choisis entre les groupes alkyle inférieur de 1 à 5 atomes de carbone, les atomes d'halogène, les groupes alcoxy inférieur de 1 à 5 atomes de carbone, et le groupe phényle, 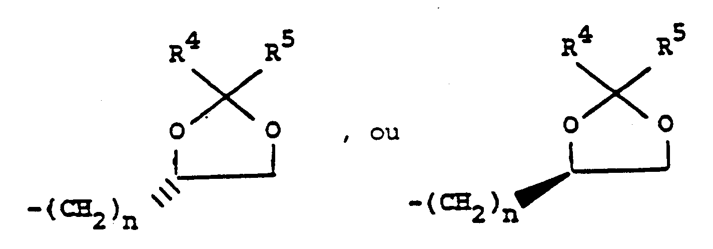 où n est un nombre entier de 1 à 4 et R⁴ et R⁵ sont choisis indépendamment entre l'hydrogène et les groupes alkyle inférieur de 1 à 5 atomes de carbone; et
R² est un groupe alkyle inférieur de 1 à 5 atomes de carbone, ou bien les deux groupes R² sont réunis par un groupe alkylène de 2 ou 3 atomes de carbone, pour la préparation d'un composé de formule 1 tel qu'il est défini dans la revendication 1.

9. Utilisation selon la revendication 8, dans laquelle R¹ est choisi entre

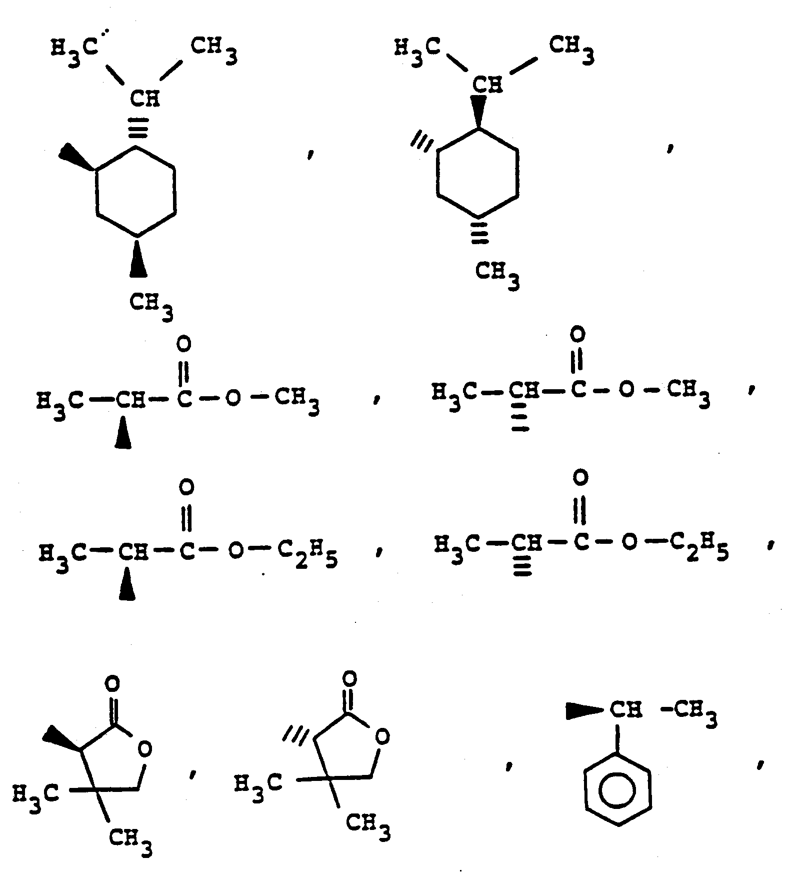

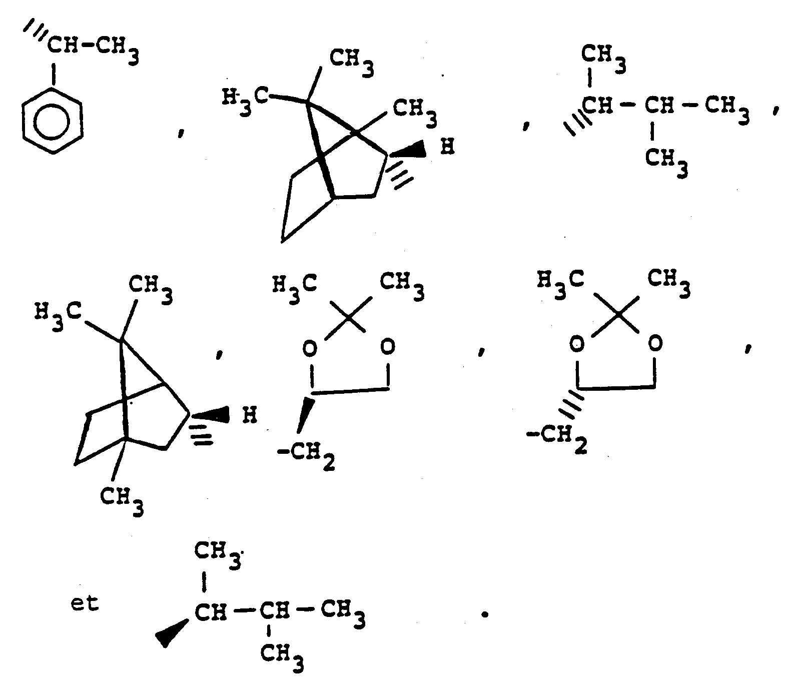

10. Utilisation selon la revendication 8, dans laquelle R¹ est 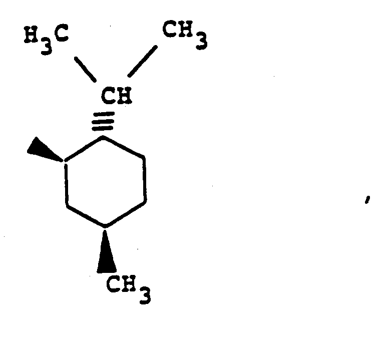 et
R² est le groupe méthyle, dont le nom est ester di-(-)-mentylique de l'acide (1S-trans)-3,3-diméthoxy-1,2-cyclobutanedicarboxylique.
